# EUROPEAN PATENT APPLICATION

(11) **EP 4 177 336 A1**
(43) Date of publication of application: **10.05.2023**
(21) Application number: 22194549.6
(22) Date of filing: 18.12.2015
(51) Int. Cl.: C12N 5/00, C12N 5/071, C12N 5/0781

(54) **B CELLS FOR IN VIVO DELIVERY OF THERAPEUTIC AGENTS**

(30) Priority: 19.12.2014 US 201462094794 P; 30.01.2015 US 201562110063 P
(62) Divisional of application: 15871244.8
(71) Applicant: Immusoft Corporation, Seattle, WA 98121 (US)
(72) Inventor: SCHOLZ, Matthew Rein, Seattle, WA 98121 (US); HERBIG, Eric J., Seattle, WA 98121 (US); XU, Mei, Seattle, WA 98121 (US); DE LAAT, Rian, Seattle, WA 98121 (US)
(74) Representative: Schiweck Weinzierl Koch Patentanwälte Partnerschaft mbB

(57) **Abstract**

The present disclosure relates to genetically modified B cells, including memory cells, differentiated to plasmablasts or plasma cells useful for long term *in vivo* expression of a transgene, such as a specific antibody or other protein therapeutic. Also disclosed are methods of producing the cells and methods of treatment.

## Description

### CROSS-REFERENCE TO RELATED APPLICATIONS

This application claims priority to U.S. Provisional Application No. 62/110,063 filed on January 30, 2015 and U.S. Provisional Application No. 62/094,794 filed on December 19, 2014, which applications are incorporated by reference herein in their entirety.

### BACKGROUND

### Technical Field

The present disclosure relates to the use of B cells for long term *in vivo* delivery of a therapeutic agent, such as an antigen-specific antibody or protein (*e*.*g*., an enzyme).

### Description of the Related Art

After leaving the bone marrow, a B cell acts as an antigen presenting cell (APC) and internalizes antigens. Antigen is taken up by the B cell through receptor-mediated endocytosis and processed. Antigen is processed into antigenic peptides, loaded onto MHC II molecules, and presented on the B cell extracellular surface to CD4+ T helper cells. These T cells bind to the MHC II/antigen molecule and cause activation of the B cell. Upon stimulation by a T cell, the activated B cell begins to differentiate into more specialized cells. Germinal center B cells may differentiate into long-lived memory B cells or plasma cells. Further, secondary immune stimulation may result in the memory B cells giving rise to additional plasma cells. The formation of plasma cells from either memory or non-memory B cells is preceded by the formation of precursor plasmablasts that eventually differentiate into plasma cells, which produce large volumes of antibodies (see e.g., Trends Immunol. 2009 June; 30(6): 277-285; Nature Reviews, 2005, 5:231 -242). Plasmablasts secrete more antibodies than B cells, but less than plasma cells. They divide rapidly, and they continue to internalize antigens and present antigens to T cells. Plasmablasts have the capacity to migrate to sites of chemokine production (*e*.*g*. in bone marrow) whereby they may differentiate into long-lived plasma cells. Ultimately, a plasmablast may either remain as a plasmablast for several days and then die or irrevocably differentiate into a mature, fully differentiated plasma cell. Specifically, plasmablasts that are able home to tissues containing plasma cell survival niches (*e*.*g*., in bone marrow) are able to displace resident plasma cells in order to become long lived plasma cells, which may continue to secrete high levels of proteins for years.

Current cell therapy methods (*e*.*g*., adoptive transfer of stem cells) are promising strategies for the treatment of various diseases and disorders; however, there still remains a need in the art for the long term treatment for many chronic diseases and disorders. The present disclosure provides differentiated B cell compositions for long term *in vivo* expression of a transgene and methods for producing the B cell compositions and use in prophylactic and therapeutic applications. The present disclosure provides these and other advantages as described in the detailed description.

### SUMMARY OF THE INVENTION

One aspect of the present invention provides a method of producing a modified B cell composition comprising (a) isolating pan-B cells, memory B cells, switched memory B cells, or plasma cells from a sample, thereby obtaining an isolated B cell population, (b) culturing the isolated B cell population *in vitro* with one or more B cell activating factors, thereby obtaining an expanded B cell population, (c) transfecting the expanded B cell population with a transgene, and (d) differentiating the expanded B cell population *in vitro* with one or more B cell activating factors, thereby obtaining a modified B cell composition. In one embodiment, the transfecting step further comprises enriching the expanded B cell population using a selectable marker. In a further embodiment, the selectable marker is selected from the group consisting of a fluorescent marker protein, a drug resistance factor, and a surface marker.

In one embodiment, the isolated B cell population is CD20+, CD27+, and CD138-. In another embodiment, the isolated B cell population is CD20+ and IgG+. In one embodiment, the isolated B cell population is CD20-, CD38- and CD138-. In one embodiment, the sample is whole blood or peripheral blood mononuclear cells (PBMCs). In one embodiment, the isolating step comprises 1) depleting CD3+ and CD56+ cells and 2) enriching for CD27+ cells.

In one embodiment, the one or more B cell activating factors are selected from the group consisting of CD40L, IFN-α, IFN-δ, IL-2, IL-4, IL-6, IL-10, IL-15, IL-21 and p-ODN. In a further embodiment, the CD40L is sCD40L-his. In one embodiment, the one or more B cell activating factors of the culturing step comprise CD40L, IL-2, IL-4 and IL-10. In one embodiment, the one or more B cell activating factors of the culturing step comprise CD40L, IL-2, IL-4, IL-10, IL-15 and IL-21. In one embodiment, the culturing step comprises a CD40L crosslinking agent. In one embodiment, feeder cells are absent from the culturing step.

In one embodiment, the expanded B cell population is migratory. In one embodiment, cells of the expanded B cell population migrate toward CXCL12. In one embodiment, at least 20% of the cells of the expanded B cell population are migratory.

In another embodiment, the one or more B cell activating factors of the differentiating step comprise CD40L, CpG, IFN-α, IFN-δ, IL-2, IL-6, IL-10, and IL-15.

In one embodiment, the transfecting comprises electroporation, lipofection, cell squeezing or viral transduction. In a further embodiment, the viral transduction comprises a retroviral vector. In one embodiment, the retroviral vector is a lentiviral vector.

In another embodiment, the transfecting comprises a non-viral vector. In a further embodiment, the non-viral vector is selected from the group consisting of a transposon, a zinc-finger nuclease, a transcription activator-like effector nuclease, a clustered regularly interspaced short palindromic repeat, a minicircle, a DNA replicon, an RNA replicon, an artificial chromosome, a plasmid, a mini-intronic plasmid, a nanoplasmid, a cosmid, and a bacteriophage. In one embodiment, the non-viral vector is a transposon. In another embodiment, the non-viral vector is a minicircle, a mini-intronic plasmid, or a nanoplasmid.

In one embodiment, the transgene encodes an antigen-specific antibody, or antigen-binding fragment thereof, a fusion protein, or a therapeutic protein. In a further embodiment, the therapeutic protein is selected from the group consisting of a cell surface receptor, a secreted protein, a signaling molecule, an antigenic fragment, an enzyme, a clotting factor, and an adhesion molecule. In another embodiment, the antibody is an anti-HIV antibody, an anti-RNA antibody, or an antibody that binds a protein involved in immune regulation. In one embodiment, the cells of the modified B cell composition are CD20-, CD38+ and CD138+. In one embodiment, the cells of the modified B cell composition are CD20-, CD38+ and CD138-.

Another aspect of the invention provides a modified B cell composition produced according to the method described above. In one embodiment, a majority of the cells of the composition survive for 10 or more days following *in vivo* administration. In another embodiment, a majority of the cells of the composition survive for 30 or more days following *in vivo* administration.

### BRIEF DESCRIPTION OF THE DRAWINGS

Figure 1 is a line graph that shows expansion of B cells in culture as measured by automated cell counting. B cells were expanded using a combination of IL-2, IL-10, CpG and CD40L. Cells can be further expanded in culture if passaged upon reaching a cell density of 5 × 10⁶ cells/ml.
Figure 2 is a series of flow cytometry dot plots that show differentiation of B cells on Day 0, Day 6, and Day 9 of *in vitro* culture using a three stage culture system designed to produce CD20-, CD38+ activated B cells. CD20 is shown on the y-axis, and CD38 is shown on the x-axis.

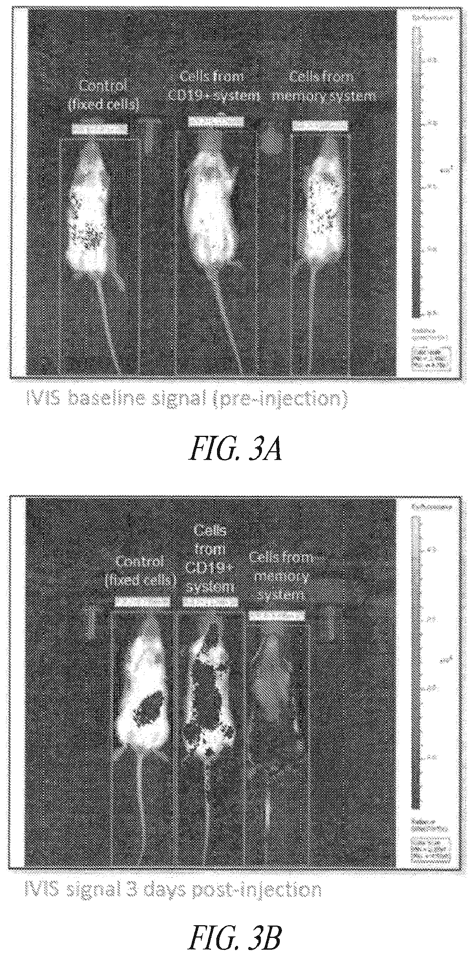

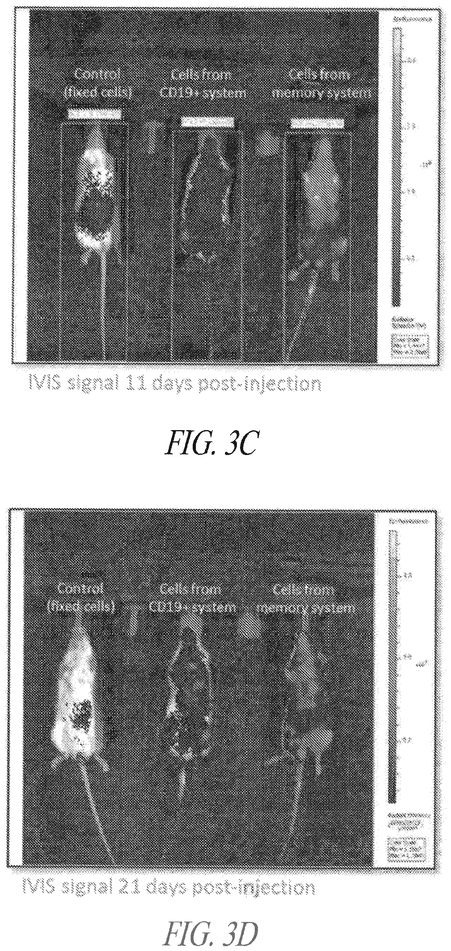

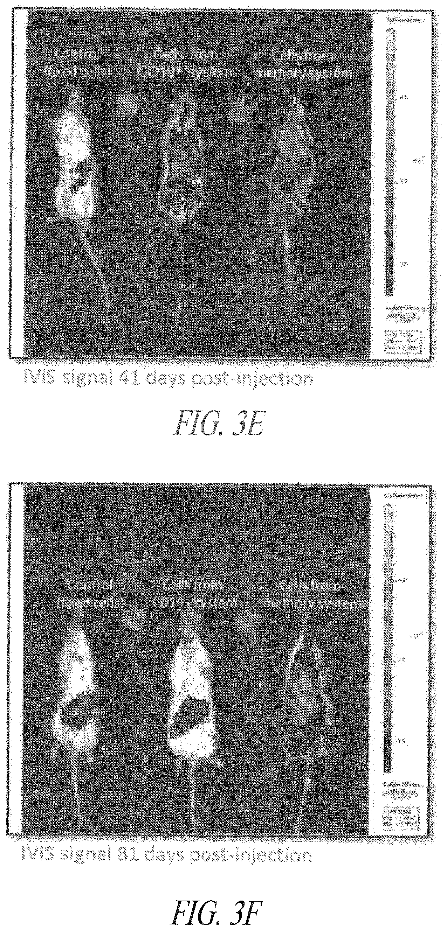

Figure 3 is a series of *In Vivo* Imaging System^{®} (IVISO) images that show the relative amount and location of cells injected into mice pre-injection (Figure 3A) and at 3, 11, 21, 41 and 81 days post-injection (Figures 3B-3F, respectively). Control mice received fixed cells, one group of mice received pan-B cells (CD19+) that were activated using a three stage *in vitro* culture system, and the third group of mice received cells generated from memory B cells that were activated using the three stage *in vitro* culture system. The cells were stained with a near infrared fluorescent dye, DiR, prior to injection, and the images show the resulting fluorescent signal.
Figure 4 is a line graph that shows the engraftment and persistence of human primary B cells in NSG mice depicted in Figure 3. Data from control mice that received fixed cells and mice injected with plasmablasts derived from memory B cells and pan-B cells (CD19+).
Figure 5A is a flow cytometry dot plot that shows control memory B cells (not transduced).
Figure 5B is a flow cytometry dot plot that shows memory B cells transduced with GFP encoding lentiviral vectors.
Figure 6 is a bar graph that shows production of IDUA by B cells as assessed by fluorometric assay. B cells were modified to stably express IDUA using the Sleeping Beauty Transposon System. For a negative control, cells were modified to express GFP. IDUA levels were measured in tissue culture media on day 10 of culture using an enzyme assay. Based on the results from multiple experiments, stably modified B cells were found to produce an average of 0.09 pMol of IDUA/cell/hour.
Figure 7 is a bar graph that shows production of VRC01 transduced B cells (VRC01) as determined by a gp120 binding ELISA. After 10 days in culture, the differentiated B cells produced an average of 1.47 pg VRC01/cell/day. VRC01 was not detected from control B cells.
Figure 8 is a series of flow cytometry dot plots that show transfection of memory B cells using the 4D-Nucleofactor^{™} System (Lonza). Control cells were electroporated with an empty transposon vector (Figure 8A). Figure 8B shows cells that were electroporated with a transposon harboring GFP in the absence of transposase, and Figure 8C shows cells that were electroporated with a transposon harboring GFP along with the transposase SB100x.
Figure 9 is a series of flow cytometry dot plots that show differentiation of pan (CD27+) memory B cells in an IL-21 containing culture system. Samples from days 0, 3, 6 and 9 of *in vitro* culture are shown from top to bottom. Forward and side scatter gating for lymphocytes is shown to the left of each resulting plot of CD20 (y-axis) vs. CD38 (x-axis) expression.
Figure 10 shows two flow cytometry dot plots for expression of CD20 (y-axis) and CD38 (x-axis) by switched memory B cells (top panel) and pan (CD27+) memory B cells (lower panel) following *in vitro* differentiation in an IL-21 containing culture system.
Figure 11 shows two flow cytometry histograms for expression of CD138 (x-axis) after differentiation of switched memory B cells (top panel) and pan (CD27+) memory B cells (lower panel) in an IL-21 containing culture system.
Figure 12 is a line graph that shows cell proliferation using two cell culture conditions. Culture A utilizes CD40L, IL-2, IL-4, and IL-10, and Culture B utilizes CD40L, IL-2, IL-4, IL-10, IL-15, and IL-21.
Figure 13 is a line graph that shows the effect of IL-15 and IL-21 separately and in combination on pan-B cell proliferation *in vitro.*
Figure 14 is a line graph that shows the proliferation of pan-B cells over 10 days in culture with CD40L, a CD40L crosslinking agent, IL-2, IL-4, IL-10, IL-15, and IL-21.
Figure 15 is a bar graph (top panel) that shows the migration of B cells toward a chemoattractant, CXCL12. The bottom panel is a flow diagram of the assay.

### DETAILED DESCRIPTION

The present invention is based, in part, on the discovery that utilizing memory B cells, rather than naive B cells, as the starting cell population and the system described herein for activation, differentiation and expansion results in a cell composition that demonstrates improved *in vivo* survival. Additionally, processes for the expansion, or proliferation, of naive and memory B cells *in vitro* in the absence of feeder, or helper, cells were discovered. The B cell expansion methods described herein enable the large scale manufacture of B cell compositions. The B cell compositions described herein are useful for *in vivo* delivery and expression of therapeutic agents, including, *e*.*g*., antigen-specific antibodies and other proteins. In particular, the B cell compositions described herein are useful for long term *in vivo* delivery and expression of therapeutic agents. The present disclosure relates generally to *in vitro* culture and production methods for B cells under conditions so as to differentiate B cells into plasmablasts and plasma cells prior to *in vivo* administration, to elicit production of therapeutic proteins from these cells, and/or to achieve sufficient enrichment and number of cells producing therapeutic proteins.

As used herein, the phrases "long term *in vivo* survival" and "long term survival" refer to the survival of the cells of a B cell composition described herein for 10 or more days post administration in a subject. Long term survival may be measured in days, weeks, or even years. In one embodiment, a majority of the cells of a B cell composition survive *in vivo* for 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more days post-administration. In one embodiment, a majority of the cells of a B cell composition survive *in vivo* for 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52 or more weeks post-administration. In another embodiment, the cells of a B cell composition survive *in vivo* for 1, 1.5, 2, 2.5, 3, 3.5, 4, 4.5, 5, 6, 7, 8, 9, 10, 11, 12, 13, 14, 15, 16, 17, 18, 19, 20, 25, 30 or more years. Additionally, while the cells of a B cell composition described herein may survive *in vivo* for 10 or more days, it is understood that a majority of the cells of a B cell composition survive *in vivo* for 1, 2, 3, 4, 5, 6, 7, 8, 9 or more days post-administration. Accordingly, it is contemplated that B cell compositions described herein are useful for short-term treatment (*e*.*g*., 4 days) and long-term treatment (*e*.*g*., 30 or more days) methods.

### Isolating B cells

The B cells used in the methods described herein are isolated pan B cells, memory B cells, plasmablasts, and/or plasma cells. In one embodiment, the isolated B cells are memory B cells. In another embodiment, the isolated B cells are naive B cells. In one embodiment, the isolated B cells are pan B cells. However, one of ordinary skill in the art will readily appreciate that the vectors and methods of the disclosure may be applied to other cell types.

Terminally differentiated plasma cells do not express common pan-B cell markers, such as CD20, and express relatively few surface antigens. Some terminally differentiated plasma cells also do not express CD19. Plasma cells express CD38, CD78, CD138 and interleukin-6 receptor (IL-6R) and lack expression of CD45, and these markers can be used, e.g., by flow cytometry, to identify plasma cells. CD27 is also a good marker for plasma cells as naive B cells are CD27-, memory B cells are CD27+ and plasma cells are CD27++. Memory B cell subsets may also express surface IgG, IgM and IgD, whereas plasma cells do not express these markers on the cell surface. CD38 and CD138 are expressed at high levels on plasma cells (See Wikipedia, The Free Encyclopedia., "Plasma cell" Page Version ID: 404969441 ; Date of last revision: 30 December 2010 09:54 UTC, retrieved January 4, 2011 ; See also: Jourdan et al. Blood. 2009 Dec 10;114(25):5173-81; Trends Immunol. 2009 June; 30(6): 277-285; Nature Reviews, 2005, 5:231 - 242; Nature Med. 2010, 16:123-129; Neuberger, M. S.; Honjo, T.; Alt, Frederick W. (2004). Molecular biology of B cells. Amsterdam: Elsevier, pp. 189-191 ; Bertil Glader; Greer, John G.; John Foerster; Rodgers, George G.; Paraskevas, Frixos (2008). Wintrobe's Clinical Hematology, 2-Vol. Set. Hagerstwon, MD: Lippincott Williams & Wilkins. pp. 347; Walport, Mark; Murphy, Kenneth; Janeway, Charles; Travers, Paul J. (2008). Janeway's immunobiology. New York: Garland Science, pp. 387-388; Rawstron AC (May 2006). "Immunophenotyping of plasma cells". Curr Protoc Cytom).

"Quiescent", as used herein, refers to a cell state wherein the cell is not actively proliferating.

"Activated", as used herein, refers to a cell state wherein the cell is actively proliferating and/or producing cytokines in response to a stimulus.

The terms "differentiate" and "differentiated", as used herein, refer to changes in the phenotype of a cell from one cell type or state to another cell type or state. For example, a memory B cell that transitions to a plasma cell is differentiated.

Prior to differentiation and transfection, a source of B cells is obtained from a subject. The term "subject" is intended to include living organisms in which an adaptive immune response can be elicited (e.g., mammals). Examples of subjects include humans, dogs, cats, mice, rats, and transgenic species thereof. B cells can be obtained from a number of sources, including peripheral blood mononuclear cells (PBMCs), bone marrow, lymph node tissue, cord blood, tissue from a site of infection, spleen tissue, and tumors. In a preferred embodiment, the source of B cells is PBMCs. In certain embodiments of the present disclosure, any number of B cell lines available in the art may be used.

In certain embodiments of the methods described herein, B cells can be obtained from a unit of blood collected from a subject using any number of techniques known to the skilled artisan, such as FICOLL^{™} (copolymers of sucrose and epichlorohydrin that may be used to prepare high density solutions) separation. In one preferred embodiment, cells from the circulating blood of an individual are obtained by apheresis or leukapheresis. The apheresis product typically contains lymphocytes, including T cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing steps. In one embodiment of the methods described herein, the cells are washed with phosphate buffered saline (PBS). In an alternative embodiment, the wash solution lacks calcium and may lack magnesium or may lack many if not all divalent cations. As those of ordinary skill in the art would readily appreciate a washing step may be accomplished by methods known to those in the art, such as by using a semi-automated "flow-through" centrifuge (for example, the Cobe 2991 cell processor) according to the manufacturer's instructions. After washing, the cells may be resuspended in a variety of biocompatible buffers, such as, for example, PBS. Alternatively, the undesirable components of the apheresis sample may be removed and the cells directly resuspended in culture media.

B cells may be isolated from peripheral blood or leukapheresis using techniques known in the art. For example, PBMCs may be isolated using FICOLL^{™} (Sigma-Aldrich, St Louis, MO) and CD19+ B cells purified by negative or positive selection using any of a variety of antibodies known in the art, such as the Rosette tetrameric complex system (StemCell Technologies, Vancouver, Canada) or MACS^{™} MicroBead Technology (Miltenyi Biotec, San Diego, CA). In certain embodiments, memory B cells are isolated as described by Jourdan et al., (Blood. 2009 Dec 10; 114(25):5173-81). For example, after removal of CD2+ cells using anti-CD2 magnetic beads, CD19+ CD27+ memory B cells can be sorted by FACS. Bone marrow plasma cells (BMPCs) can be purified using anti-CD138 magnetic microbeads sorting or other similar methods and reagents.

Other isolation kits are commercially available, such as R&D Systems' MagCellect Human B Cell Isolation Kit (Minneapolis, MN). In certain embodiments, resting B cells may be prepared by sedimentation on discontinuous Percoll gradients, as described in (Defranco et al., (1982) J. Exp. Med. 155:1523).

In one embodiment, PBMCs are obtained from a blood sample using a gradient based purification (*e*.*g*., FICOLL^{™}). In another embodiment, PBMCs are obtained from aphersis based collection. In one embodiment, B cells are isolated from PBMCs by isolating pan B cells. The isolating step may utilize positive and/or negative selection. In one embodiment, the negative selection comprises depleting T cells using anti-CD3 conjugated microbeads, thereby providing a T cell depleted fraction. In a further embodiment, memory B cells are isolated from the pan B cells or the T cell depleted fraction by positive selection for CD27.

In one embodiment, switched memory B cells are obtained. "Switched memory B cell" or "switched B cell," as used herein, refers to a B cell that has undergone isotype class switching. In one embodiment, switched memory B cells are positively selected for IgG. In another embodiment, switched memory B cells are obtained by depleting IgD and IgM expressing cells.

In a further embodiment the promoter sequence from a gene unique to memory B cells, such as, e.g., the CD27 gene (or other gene specific to memory B cells and not expressed in naive B cells) is used to drive expression of a selectable marker such as, e.g., mutated dihydrofolate reductase allowing for positive selection of the memory B cells in the presence of methotrexate. In another embodiment T cells are depleted using CD3 or by addition of cyclosporin. In another embodiment, CD138+ cells are isolated from the pan B cells by positive selection. In yet another embodiment, CD138+ cells are isolated from PBMCs by positive selection. In another embodiment, CD38+ cells are isolated from the pan B cells by positive selection. In yet another embodiment, CD38+ cells are isolated from PBMCs by positive selection. In one embodiment, CD27+ cells are isolated from PBMCs by positive selection. In another embodiment, memory B cells and/or plasma cells are selectively expanded from PBMCs using *in vitro* culture methods available in the art.

### Culturing B Cells In Vitro

The present disclosure provides methods of culturing B cells, such as memory B cells and/or naive B cells, in order to activate and differentiate the B cells into plasma cells or plasmablasts or both. As would be recognized by the skilled person, plasma cells may be identified by cell surface protein expression patterns using standard flow cytometry methods. For example, terminally differentiated plasma cells express relatively few surface antigens, and do not express common pan-B cell markers, such as CD20. Some terminally differentiated plasma cells also do not express CD19. Instead, plasma cells may be identified by expression of CD38, CD78, CD138, and IL-6R and lack of expression of CD45. CD27 may also be used to identify plasma cells as naive B cells are CD27-, memory B cells are CD27+ and plasma cells are CD27++. Plasma cells express high levels of CD38 and CD138.

In one embodiment, following certain steps of the culture methods described herein the isolated cells are CD20-, CD38-, CD138- memory B cells. In one embodiment, the isolated cells are CD20-, CD38+, CD138+ plasma cells. In one embodiment, the cells are activated and have a cell surface phenotype of CD20-, CD38-, CD138-, CD27+. In one embodiment, the isolated cells are CD20-, CD38+, CD138-, and CD27+.

In one embodiment, the B cells are contacted with one or more B cell activating factors, e.g., any of a variety of cytokines, growth factors or cell lines known to activate and/or differentiate B cells (see e.g., Fluckiger, et al. Blood 1998 92: 4509-4520; Luo, et al., Blood 2009 1 13: 1422-1431 ). Such factors may be selected from the group consisting of, but not limited to, IL-1, IL-2, IL-3, IL-4, IL-5, IL-6, IL-7, IL-8, IL-9, IL-10, IL-1 1 , IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, and IL-35, IFN-γ, IFN-α, IFN-β, IFN-δ, C type chemokines XCL1 and XCL2, C-C type chemokines (to date including CCL1 -CCL28) and CXC type chemokines (to date including CXCL1 -CXCL17), and members of the TNF superfamily (*e*.*g*., TNF-α, 4-1 BB ligand, B cell activating factor (BLyS), FAS ligand, sCD40L (including multimeric versions of sCD40L; *e*.*g*., histidine-tagged soluble recombinant CD40L in combination with anti-poly-histidine mAb to group multiple sCD40L molecules together), Lymphotoxin, OX40L, RANKL, TRAIL), CpG, and other toll like receptor agonists (*e*.*g*., CpG).

B cell activating factors may be added to *in vitro* cell cultures at various concentrations to achieve the desired outcome (*e*.*g*., expansion or differentiation). In one embodiment, a B cell activating factor is utilized in expanding the B cells in culture. In one embodiment, a B cell activating factor is utilized in differentiating the B cells in culture. In another embodiment, the B cell activating factor is utilized in both expanding and differentiating the B cells in culture. In one embodiment, the B cell activating factor is provided at the same concentration for expanding and differentiating. In another embodiment, the B cell activating factor is provided at a first concentration for expanding and at a second concentration for differentiating. It is contemplated that a B cell activating factor may be 1) utilized in expanding the B cells and not in differentiating the B cells, 2) utilized in differentiating the B cells and not in expanding the B cells, or 3) utilized in expanding and differentiating the B cells.

For example, B cells are cultured with one or more B cell activating factors selected from CD40L, IL-2, IL-4, and IL-10 for expansion of the B cells. In one embodiment, the B cells are cultured with 0.25-5.0 µg/ml CD40L. In one embodiment, the concentration of CD40L is 0.5 µg/ml. In one embodiment a crosslinking agent (such as an anti-HIS antibody in combination with HIS-tagged CD40L) is used to create multimers of CD40L. In one embodiment molecules of CD40L are covalently linked or are held together using protein multimerization domains (*e*.*g*., the Fc region of an IgG or a leucine zipper domain). In one embodiment CD40L is conjugated to beads. In one embodiment CD40L is expressed from feeder cells. In another embodiment, feeder cells are absent. In one embodiment, the B cells are cultured with 1-10 ng/ml IL-2. In one embodiment, the concentration of IL-2 is 5 ng/ml. In one embodiment, the B cells are cultured with 1-10 ng/ml IL-4. In one embodiment, the concentration of IL-4 is 2 ng/ml. In one embodiment, the B cells are cultured with 10-100 ng/ml IL-10. In one embodiment, the concentration of IL-10 is 40 ng/ml.

In one embodiment, B cells are cultured with one or more B cell activating factors selected from CD40L, IL-2, IL-4, IL-10, IL-15 and IL-21 for expansion of the B cells. In one embodiment, the B cells are cultured with 0.25-5.0 µg/ml CD40L. In one embodiment, the concentration of CD40L is 0.5 µg/ml. In one embodiment a crosslinking agent (such as an anti-HIS antibody in combination with HIS-tagged CD40L) is used to create multimers of CD40L. In one embodiment molecules of CD40L are covalently linked or are held together using protein multi-merization domains (e.g., the Fc region of an IgG or a leucine zipper domain). In one embodiment CD40L is conjugated to beads. In one embodiment CD40L is expressed from feeder cells. In one embodiment, the B cells are cultured with 1-10 ng/ml IL-2. In one embodiment, the concentration of IL-2 is 5 ng/ml. In one embodiment, the B cells are cultured with 1-10 ng/ml IL-4. In one embodiment, the concentration of IL-4 is 2 ng/ml. In one embodiment, the B cells are cultured with 10-100 ng/ml IL-10. In one embodiment, the concentration of IL-10 is 40 ng/ml. In one embodiment, the B cells are cultured with 50-150 ng/ml IL-15. In one embodiment, the concentration of IL-15 is 100 ng/ml. In one embodiment, the B cells are cultured with 50-150 ng/ml IL-21. In one embodiment, the concentration of IL-21 is 100 ng/ml. In one embodiment, the B cells are cultured with CD40L, IL-2, IL-4, and IL-10. In a particular embodiment, the B cells are cultured with CD40L, IL-2, IL-4, IL-10, IL-15 and IL-21 for expansion of the B cells. In another embodiment, the B cells are cultured with CD40L, IL-2, IL-4, IL-10, IL-15, IL-21, and a CD40L crosslinking agent (*e*.*g*., a CD40L cross-linking antibody). In one embodiment, the CD40L is HIS-tagged CD40L, and the CD40L cross-linking antibody is an anti-HIS antibody.

In another example, B cells are cultured with one or more B cell activating factors selected from CD40L, IFN-α and IFN-δ mix, IL-2, IL-6, IL-10, IL-15, IL-21, and P-class CpG oligodeoxynucleotides (p-ODN) for differentiation of the B cells. In one embodiment, the B cells are cultured with 25-75 ng/ml CD40L. In one embodiment, the concentration of CD40L is 50 ng/ml. In one embodiment, the B cells are cultured with 250-750 U/ml IFN-α and IFN-δ mix. In one embodiment the concentration of the IFN-α and IFN-δ mix is 500 U/ml. In one embodiment, the B cells are cultured with 5-50 U/ml IL-2. In one embodiment the concentration of IL-2 is 20 U/ml. In one embodiment, the B cells are cultured with 25-75 ng/ml IL-6. In one embodiment, the concentration of IL-6 is 50 ng/ml. In one embodiment, the B cells are cultured with 10-100 ng/ml IL-10. In one embodiment, the concentration of IL-10 is 50 ng/ml. In one embodiment, the B cells are cultured with 1-20 ng/ml IL-15. In one embodiment, the concentration of IL-15 is 10 ng/ml. In one embodiment, the B cells are cultured with 10-100 ng/ml IL-21. In one embodiment, the concentration of IL-21 is 50 ng/ml. In one embodiment, the B cells are cultured with 1-50 µg/ml p-ODN. In one embodiment, the concentration of p-ODN is 10 µg/ml.

In one embodiment, B cells are contacted or cultured on feeder cells. In one embodiment, the feeder cells are a stromal cell line, e.g., murine stromal cell line S17 or MS5. In another embodiment, isolated CD19+ cells are cultured with one or more B cell activating factor cytokines, such as IL-10 and IL-4, in the presence of fibroblasts expressing CD40-ligand (CD40L, CD154). In one embodiment, CD40L is provided bound to a surface such as tissue culture plate or a bead. In another embodiment, purified B cells are cultured, in the presence or absence of feeder cells, with CD40L and one or more cytokines or factors selected from IL-10, IL-4, IL-7, p-ODN, CpG DNA, IL-2, IL-15, IL6, IFN-α, and IFN-δ.

In another embodiment, B cell activating factors are provided by transfection into the B cell or other feeder cell. In this context, one or more factors that promote differentiation of the B cell into an antibody secreting cell and/or one or more factors that promote the longevity of the antibody producing cell may be used. Such factors include, for example, Blimp-1, TRF4, anti-apoptotic factors like Bcl-xl or Bcl5, or constitutively active mutants of the CD40 receptor. Further, factors which promote the expression of downstream signaling molecules such as TNF receptor-associated factors (TRAFs) may also be used in the activation/differentiation of the B cells. In this regard, cell activation, cell survival, and antiapoptotic functions of the TNF receptor superfamily are mostly mediated by TRAF1-6 (see *e*.*g*., R.H. Arch, et al., Genes Dev. 12 (1998), pp. 2821-2830). Downstream effectors of TRAF signaling include transcription factors in the NF- κB and AP-1 family which can turn on genes involved in various aspects of cellular and immune functions. Further, the activation of NF-κB and AP-1 has been shown to provide cells protection from apoptosis via the transcription of antiapoptotic genes.

In another embodiment, Epstein Barr virus (EBV)-derived proteins are used for the activation and/or differentiation of B cells or to promote the longevity of the antibody producing cell. EBV-derived proteins include but are not limited to, EBNA-1, EBNA-2, EBNA-3, LMP-1, LMP-2, EBER, miRNAs, EBV-EA, EBV-MA, EBV-VCA and EBV-AN.

In certain embodiments, contacting the B cells with B cell activation factors using the methods provided herein leads to, among other things, cell proliferation (*i*.*e*., expansion), modulation of the IgM+ cell surface phenotype to one consistent with an activated mature B cell, secretion of Ig, and isotype switching. CD19+ B cells may be isolated using known and commercially available cell separation kits, such as the MiniMACS^{™} cell separation system (Miltenyi Biotech, Bergisch Gladbach, Germany). In certain embodiments, CD40L fibroblasts are irradiated before use in the methods described herein. In one embodiment, B cells are cultured in the presence of one or more of IL-3, IL-7, Flt3 ligand, thrombopoietin, SCF, IL-2, IL-10, G-CSF and CpG. In certain embodiments, the methods include culturing the B cells in the presence of one or more of the aforementioned factors in conjunction with transformed stromal cells (*e*.*g*., MS5) providing a low level of anchored CD40L and/or CD40L bound to a plate or a bead.

In one embodiment, contacting the B cells with B cell activating factors using the methods provided herein induces the B cells to be migratory. Migratory B cells are able to, for example, migrate to survival niches, complete differentiation, receive signals supporting long-term survival following *in vivo* administration, and move to sites of inflammation. For example, CXCL12 draws plasmablasts to the bone marrow, which is important for their long term survival. Similarly, CXCL13, for example, draws B cells towards sites of inflammation. Expanded and/or activated B cells may be migratory. In one embodiment, an expanded B cell population is migratory. In one embodiment, cells of the expanded B cell population migrate toward CXCL12. In one embodiment, cells of the expanded B cell population migrate toward CXCL13. In one embodiment, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or even 100% of the cells of a B cell population (e.g., an expanded B cell population) are migratory. In one embodiment, a population of B cells is migratory when at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or even 100% of the cells migrate toward a chemoattractant. In one embodiment, the chemoattractant is CXCL12. In one embodiment, the chemoattractant is CXCL13.

As discussed above, B cell activating factors induce expansion, proliferation, or differentiation of B cells. Accordingly, B cells are contacted with one or more B cell activating factors listed above to obtain an expanded cell population. A cell population may be expanded prior to transfection. Alternatively, or additionally, a cell population may be expanded following transfection. In one embodiment, expanding a B cell population comprises culturing cells with IL-2, IL-4, IL-10 and CD40L (see *e*.*g*., Neron et al. PLoS ONE, 2012 7(12):e51946). In one embodiment, expanding a B cell population comprises culturing cells with IL-2, IL-10, CpG, and CD40L. In one embodiment, expanding a B cell population comprises culturing cells with IL-2, IL-4, IL-10, IL-15, IL-21 and CD40L. In one embodiment, expanding a B cell population comprises culturing cells with IL-2, IL-4, IL-10, IL-15, IL-21, CD40L, and a CD40L crosslinking agent in the absence of feeder, or helper, cells.

In another embodiment, expansion of a B cell population is induced and/or enhanced by a transgene introduced into the B cells. For example, a B cell that contains a recombinant receptor or an engineered receptor that induces a cell signaling pathway (*e*.*g*., signaling downstream of CD40) upon binding its ligand (*e*.*g*., a soluble ligand or a cell surface expressed ligand). In one embodiment, a B cell overexpresses CD40 due to expression of a CD40 transgene. In another embodiment, a B cell expresses an engineered receptor, including, *e*.*g*., a recombinantly engineered antibody. In one embodiment, an engineered receptor is similar to a chimeric antigen receptor (CAR) and comprises a fusion protein of an scFv and an intracellular signaling portion of a B cell receptor (*e*.*g*., CD40).

In one embodiment, expansion of a B cell population is induced and/or enhanced by a small molecule compound added to the cell culture. For example, a compound that binds to and dimerizes CD40 can be used to trigger the CD40 signaling pathway.

Any of a variety of culture media may be used in the present methods as would be known to the skilled person (see e.g., Current Protocols in Cell Culture, 2000-2009 by John Wiley & Sons, Inc.). In one embodiment, media for use in the methods described herein includes, but is not limited to Iscove modified Dulbecco medium (with or without fetal bovine or other appropriate serum). Illustrative media also includes, but is not limited to, IMDM, RPMI 1640, AIM-V, DMEM, MEM, a-MEM, F-12, X-Vivo 15, and X-Vivo 20. In further embodiments, the medium may comprise a surfactant, an antibody, plasmanate or a reducing agent (*e*.*g*. N-acetyl-cysteine, 2-mercaptoethanol), one or more antibiotics, and/or additives such as insulin, transferrin, sodium selenite and cyclosporin. In some embodiments, IL-6, soluble CD40L, and a cross-linking enhancer may also be used.

B cells are cultured under conditions and for sufficient time periods to achieve differentiation and activation desired. In certain embodiments, the B cells are cultured under conditions and for sufficient time periods such that 10%, 15%, 20%, 25%, 30%, 35%, 40%, 45%, 50%, 55%, 60%, 65%, 70%, 75%, 80%, 85%, 90%, 95%, or even 100% of the B cells are differentiated and/or activated as desired. In one embodiment, the B cells are activated and differentiated into a mixed population of plasmablasts and plasma cells. As would be recognized by the skilled person, plasmablasts and plasma cells may be identified by cell surface protein expression patterns using standard flow cytometry methods as described elsewhere herein, such as expression of one or more of CD38, CD78, IL-6R, CD27^{high}, and CD138 and/or lack of, or reduction of, expression of one or more of CD19, CD20 and CD45. As would be understood by the skilled person, memory B cells are generally CD20+ CD19+ CD27+ CD38- while early plasmablasts are CD20- CD19+ CD27++ CD38++. In one embodiment, the cells cultured using the methods described herein are CD20-, CD38+, CD138-. In another embodiment, the cells have a phenotype of CD20-, CD38+, CD138+. In certain embodiments, cells are cultured for 1-7 days. In further embodiments, cells are cultured 7, 14, 21 days or longer. Thus, cells may be cultured under appropriate conditions for 1, 2, 3, 4, 5, 6, 7, 8, 9, 10, 11, 12, 13, 14, 15, 16, 17, 18, 19, 20, 21, 22, 23, 24, 25, 26, 27, 28, 29, or more days. Cells are re-plated, and media and supplements may be added or changed as needed using techniques known in the art.

In certain embodiments, the B cells are cultured under conditions and for sufficient time periods such that at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the cells are differentiated and activated to produce Ig and/or to express the transgene.

The induction of B cell activation may be measured by techniques such as ³H-uridine incorporation into RNA (as B cells differentiate, RNA synthesis increases), or by ³H-thymidine incorporation, which measures DNA synthesis associated with cell proliferation. In one embodiment, interleukin-4 (IL-4) may be added to the culture medium at an appropriate concentration (*e*.*g*., about 10 ng/ml) for enhancement of B cell proliferation.

Alternatively, B cell activation is measured as a function of immunoglobulin secretion. For example, CD40L is added to resting B cells together with IL-4 (*e*.*g*., 10 ng/ml) and IL-5 (*e*.*g*., 5 ng/ml) or other cytokines that activate B cells. Flow cytometry may also be used for measuring cell surface markers typical of activated B cells. See *e*.*g*., Civin CI, Loken MR, Int'I J. Cell Cloning 987; 5:1 -16; Loken, MR, et al, Flow Cytometry Characterization of Erythroid, Lymphoid and Monomyeloid Lineages in Normal Human Bone Marrow, in Flow Cytometry in Hematology, Laerum OD, Bjerksnes R. eds., Academic Press, New York 1992; pp. 31 -42; and LeBein TW, et ai, Leukemia 1990; 4:354-358.

After culture for an appropriate period of time, such as from 2, 3, 4, 5, 6, 7, 8, 9, or more days, generally around 3 days, an additional volume of culture medium may be added. Supernatant from individual cultures may be harvested at various times during culture and quantitated for IgM and IgG1 as described in Noelle et al., (1991) J. Immunol. 146:1118-1124. In one embodiment, the culture is harvested and measured for expression of the transgene of interest using flow cytometry, enzyme-linked immunosorbent assay (ELISA), ELISPOT or other assay known in the art.

In another embodiment, ELISA is used to measure antibody isotype production, e.g., IgM, or a product of the transgene of interest. In certain embodiments, IgG determinations are made using commercially available antibodies, such as goat anti-human IgG, as capture antibody followed by detection using any of a variety of appropriate detection reagents such as biotinylated goat antihuman Ig, streptavidin alkaline phosphatase and substrate.

In certain embodiments, the B cells are cultured under conditions and for sufficient time periods such that the number of cells is 1, 10, 25, 50, 75, 100, 125, 150, 175, 200, 250, 300, 350, 400, 450, 500, 550, 600, 650, 700, 750, 800, 850, 900, 950, 1000 fold or more greater than the number of B cells at the start of culture. In one embodiment, the number of cells is 10-1000 fold greater, including consecutive integers therein, than the number of B cells at the start of culture. For example, an expanded B cell population is at least 10 fold greater than the initial isolated B cell population. In another embodiment, the expanded B cell population is at least 100 fold greater than the initial isolated B cell population. In one embodiment, the expanded B cell population is at least 500 fold greater than the initial isolated B cell population.

### Transfection of B cells

The cells of the B cell compositions described herein are transfected with a transgene. Transfection of B cells is accomplished using any of a variety of methods available in the art to introduce DNA or RNA into a B cell. Suitable techniques may include calcium phosphate transfection, DEAE-Dextran, electroporation, pressure-mediated transfection or "cell squeezing" (*e*.*g*., CellSqueeze microfluidic system, SQZ Biotechnologies), liposome-mediated transfection and transduction using retrovirus or other virus, *e*.*g*., vaccinia. See, *e*.*g*., Graham et al., 1973, Virology 52:456; Sambrook et al., 2001, Molecular Cloning, a Laboratory Manual, Cold Spring Harbor Laboratories; Davis et al., 1986, Basic Methods in Molecular Biology, Elsevier; Chu et al., 1981, Gene 13:197; US 5,124,259; US 5,297,983; US 5,283,185; US 5,661,018; US 6,878,548; US 7,799,555; US 8,551,780; and US 8,633,029. One example of a commercially available electroporation technique suitable for B cells is the Nucleofector^{™} transfection technology.

Transfection may take place prior to or during *in vitro* culture of the isolated B cells in the presence of one or more activating and/or differentiating factors described above. For example, cells are transfected on day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or 39 of *in vitro* culture. In one embodiment, cells are transfected on day 1, 2, or 3 of *in vitro* culture. In a particular embodiment, cells are transfected on day 2. For example, cells are electroporated on day 2 of *in vitro* culture for delivery of, e.g., a plasmid, a transposon, a minicircle, or a self-replicating RNA. In another embodiment, cells are transfected on day 4, 5, 6, or 7 of *in vitro* culture. In a particular embodiment, cells are transfected on day 6 of *in vitro* culture. In another embodiment, cells are transfected on day 5 of *in vitro* culture.

In one embodiment, cells are transfected prior to activation. In another embodiment, cells are transfected during activation. In one embodiment, cells are transfected after activation. In one embodiment, cells are transfected prior to differentiation. In another embodiment, cells are transfected during differentiation. In one embodiment, cells are transfected after differentiation.

In one embodiment, a non-viral vector is used to deliver DNA or RNA to pan B cells, memory B cells, and/or plasma cells. Examples of non-viral vectors include, without limitation, transposons (*e*.*g*., Sleeping Beauty transposon system), zinc-finger nucleases (ZFNs), transcription activator-like effector nucleases (TALENs), clustered regularly interspaced short palindromic repeats (CRISPRs), minicircles, DNA replicons, RNA replicons, artificial chromosomes (*e*.*g*., bacterial artificial chromosomes, mammalian artificial chromosomes, and yeast artificial chromosomes), plasmids, mini-intronic plasmids, nanoplasmids, cosmids, and bacteriophage. In one embodiment, the non-viral vector is a persistent episomal vector.

In one embodiment, a method of transfecting a B cell comprises electroporating the B cell prior to contacting the B cell with a vector. In one embodiment, cells are electroporated on day 1, 2, 3, 4, 5, 6, 7, 8, or 9 of *in vitro* culture. In one embodiment, cells are electroporated on day 2 of *in vitro* culture for delivery of a plasmid. In one embodiment, cells are transfected using a transposon on day 1, 2, 3, 4, 5, 6, 7, 8, or 9 of *in vitro* culture. In another embodiment, cells are transfected using a minicircle on day 1, 2, 3, 4, 5, 6, 7, 8, or 9 of *in vitro* culture. In one embodiment, electroporation of a Sleeping Beauty transposon takes place on day 2 of *in vitro* culture.

In one embodiment, the B cells are contacted with a vector comprising a nucleic acid of interest operably linked to a promoter, under conditions sufficient to transfect at least a portion of the B cells. In one embodiment the B cells are contacted with a vector comprising a nucleic acid of interest operably linked to a promoter, under conditions sufficient to transfect at least 5% of the B cells. In a further embodiment, the B cells are contacted with a vector under conditions sufficient to transfect at least 5%, 10%, 15%, 20%, 25%, 30%, 35%, 40%, 45%, 50%, 55%, 60%, 70%, 75%, 80%, 85%, 90%, 95%, 96%, 97%, 98%, 99% or even 100% of the B cells. In one particular embodiment, the B cells, cultured *in vitro* as described herein, are transfected, in which case the cultured B cells are contacted with a vector as described herein under conditions sufficient to transfect at least 5%, 10% 15%, 20%, 25%, 30%, 35%, 40%, 45%, 50%, 55%, 60%, 70%, 75%, 80%, 85%, 90%, 95%, 96%, 97%, 98%, 99% or even 100% of the B cells.

Certain embodiments employ viral vectors to transduce memory B cells and/or plasma cells. Examples of viral vectors include, without limitation, adenovirus-based vectors, adeno-associated virus (AAV)-based vectors, retroviral vectors, retroviral-adenoviral vectors, and vectors derived from herpes simplex viruses (HSVs), including amplicon vectors, replication-defective HSV and attenuated HSV (see, e.g., Krisky, Gene Ther. 5: 1517-30, 1998; Pfeifer, Annu. Rev. Genomics Hum. Genet. 2:177-211, 2001, each of which is incorporated by reference in its entirety).

In one embodiment, cells are transduced with a viral vector (*e*.*g*., a lentiviral vector) on day 1, 2, 3, 4, 5, 6, 7, 8, or 9 of *in vitro* culture. In a particular embodiment, cells are transduced with a viral vector on day 5 of *in vitro* culture. In one embodiment, the viral vector is a lentivirus. In one embodiment, cells are transduced with a measles virus pseudotyped lentivirus on day 1 of *in vitro* culture.

In one embodiment, B cells are transduced with retroviral vectors using any of a variety of known techniques in the art (see, *e*.*g*., Science 12 April 1996 272: 263-267; Blood 2007, 99:2342- 2350; Blood 2009, 1 13:1422-1431 ; Blood 2009 Oct 8; 1 14(15):3173-80; Blood. 2003;101 (6):2167-2174; Current Protocols in Molecular Biology or Current Protocols in Immunology, John Wiley & Sons, New York, N.Y.(2009)). Additional description of viral transduction of B cells may be found in WO 2011/085247 and WO 2014/152832, each of which is herein incorporated by reference in its entirety.

For example, PBMCs, B- or T-lymphocytes from donors and other B cell cancer cells such as B-CLLs may be isolated and cultured in IMDM medium or RPMI 1640 (GibcoBRL Invitrogen, Auckland, New Zealand) or other suitable medium as described herein, either serum-free or supplemented with serum (*e*.*g*., 5-10% FCS, human AB serum, and serum substitutes) and penicillin/streptomycin and/or other suitable supplements such as transferrin and/or insulin. In one embodiment, cells are seeded at 1 × 10⁵ cells in 48-well plates and concentrated vector added at various doses that may be routinely optimized by the skilled person using routine methodologies. In one embodiment, B cells are transferred to an MS5 cell monolayer in RPMI supplemented with 10% AB serum, 5% FCS, 50ng/ml rhSCF, 10ng/ml rhIL-15 and 5ng/ml rhIL-2 and medium refreshed periodically as needed. As would be recognized by the skilled person, other suitable media and supplements may be used as desired.

Certain embodiments relate to the use of retroviral vectors, or vectors derived from retroviruses. "Retroviruses" are enveloped RNA viruses that are capable of infecting animal cells, and that utilize the enzyme reverse transcriptase in the early stages of infection to generate a DNA copy from their RNA genome, which is then typically integrated into the host genome. Examples of retroviral vectors Moloney murine leukemia virus (MLV)-derived vectors, retroviral vectors based on a Murine Stem Cell Virus, which provides long-term stable expression in target cells such as hematopoietic precursor cells and their differentiated progeny (see, *e*.*g*., Hawley et al., PNAS USA 93:10297-10302, 1996; Keller et al., Blood 92:877-887, 1998), hybrid vectors (see, *e*.*g*., Choi, et al., Stem Cells 19:236-246, 2001), and complex retrovirus-derived vectors, such as lentiviral vectors.

In one embodiment, the B cells are contacted with a retroviral vector comprising a nucleic acid of interest operably linked to a promoter, under conditions sufficient to transduce at least a portion of the B cells. In one embodiment the B cells are contacted with a retroviral vector comprising a nucleic acid of interest operably linked to a promoter, under conditions sufficient to transduce at least 2% of the B cells. In a further embodiment, the B cells are contacted with a vector under conditions sufficient to transduce at least 2%, 3%, 4%, 5%, 10%, 15%, 20%, 25%, 30%, 35%, 40%, 45%, 50%, 55%, 60%, 70%, 75%, 80%, 85%, 90%, 95%, 96%, 97%, 98%, 99% or even 100% of the resting B cells. In one particular embodiment, the differentiated and activated B cells, cultured *in vitro* as described herein, are transduced, in which case the cultured differentiated/activated B cells are contacted with a vector as described herein under conditions sufficient to transduce at least 2%, 3%, 4%, 5%, 10% 15%, 20%, 25%, 30%, 35%, 40%, 45%, 50%, 55%, 60%, 70%, 75%, 80%, 85%, 90%, 95%, 96%, 97%, 98%, 99% or even 100% of the differentiated and activated B cells.

In certain embodiments, prior to transduction, the cells are prestimulated with Staphylococcus Aureus Cowan (SAC; Calbiochem, San Diego, CA) and/or IL-2 at appropriate concentrations known to the skilled person and routinely optimized. Other B cell activating factors (e.g., PMA), as are known to the skilled artisan and described herein may be used.

As noted above, certain embodiments employ lentiviral vectors. The term "lentivirus" refers to a genus of complex retroviruses that are capable of infecting both dividing and non-dividing cells. Examples of lentiviruses include HIV (human immunodeficiency virus; including HIV type 1, and HIV type 2), visna-maedi, the caprine arthritis-encephalitis virus, equine infectious anemia virus, feline immunodeficiency virus (FIV), bovine immune deficiency virus (BIV), and simian immunodeficiency virus (SIV). Lentiviral vectors can be derived from any one or more of these lentiviruses (see, e.g., Evans et al., Hum Gene Ther. 10:1479-1489, 1999; Case et al., PNAS USA 96:2988-2993, 1999; Uchida et al., PNAS USA 95:1 1939-1 1944, 1998; Miyoshi et al., Science 283:682-686, 1999; Sutton et al., J Virol 72:5781 -5788, 1998; and Frecha et al., Blood. 1 12:4843-52, 2008, each of which is incorporated by reference in its entirety).

It has been documented that resting T and B cells can be transduced by a VSVG-coated LV carrying most of the HIV accessory proteins (vif, vpr, vpu, and nef) (see *e*.*g*., Frecha et al., 2010 Mol. Therapy 18:1748). In certain embodiments the retroviral vector comprises certain minimal sequences from a lentivirus genome, such as the HIV genome or the SIV genome. The genome of a lentivirus is typically organized into a 5' long terminal repeat (LTR) region, the gag gene, the pol gene, the env gene, the accessory genes (e.g., nef, vif, vpr, vpu, tat, rev) and a 3' LTR region. The viral LTR is divided into three regions referred to as U3, R (repeat) and U5. The U3 region contains the enhancer and promoter elements, the U5 region contains the polyadenylation signals, and the R region separates the U3 and U5 regions. The transcribed sequences of the R region appear at both the 5' and 3' ends of the viral RNA (see, *e*.*g*., "RNA Viruses: A Practical Approach" (Alan J. Cann, Ed., Oxford University Press, 2000); O Narayan, J. Gen. Virology. 70:1617-1639, 1989; Fields et al., Fundamental Virology Raven Press., 1990; Miyoshi et al., J Virol. 72:8150-7,1998; and U.S. Pat. No. 6,013,516, each of which is incorporated by reference in its entirety). Lentiviral vectors may comprise any one or more of these elements of the lentiviral genome, to regulate the activity of the vector as desired, or, they may contain deletions, insertions, substitutions, or mutations in one or more of these elements, such as to reduce the pathological effects of lentiviral replication, or to limit the lentiviral vector to a single round of infection.

Typically, a minimal retroviral vector comprises certain 5'LTR and 3'LTR sequences, one or more genes of interest (to be expressed in the target cell), one or more promoters, and a cis-acting sequence for packaging of the RNA. Other regulatory sequences can be included, as described herein and known in the art. The viral vector is typically cloned into a plasmid that may be transfected into a packaging cell line, such as a eukaryotic cell (e.g., 293-HEK), and also typically comprises sequences useful for replication of the plasmid in bacteria.

In certain embodiments, the viral vector comprises sequences from the 5' and/or the 3' LTRs of a retrovirus such as a lentivirus. The LTR sequences may be LTR sequences from any lentivirus from any species. For example, they may be LTR sequences from HIV, SIV, FIV or BIV. Preferably the LTR sequences are HIV LTR sequences.

In certain embodiments, the viral vector comprises the R and U5 sequences from the 5' LTR of a lentivirus and an inactivated or "self-inactivating" 3' LTR from a lentivirus. A "self-inactivating 3' LTR" is a 3' long terminal repeat (LTR) that contains a mutation, substitution or deletion that prevents the LTR sequences from driving expression of a downstream gene. A copy of the U3 region from the 3' LTR acts as a template for the generation of both LTR's in the integrated provirus. Thus, when the 3' LTR with an inactivating deletion or mutation integrates as the 5' LTR of the provirus, no transcription from the 5' LTR is possible. This eliminates competition between the viral enhancer/promoter and any internal enhancer/promoter. Self-inactivating 3' LTRs are described, for example, in Zufferey et al., J Virol. 72:9873-9880, 1998; Miyoshi et al., J Virol. 72:8150-8157, 1998; and Iwakuma et al., Wro/ogy 261: 120-132, 1999, each of which is incorporated by reference in its entirety. Self-inactivating 3' LTRs may be generated by any method known in the art. In certain embodiments, the U3 element of the 3' LTR contains a deletion of its enhancer sequence, preferably the TATA box, Spl and/or NF-kappa B sites. As a result of the self-inactivating 3' LTR, the provirus that is integrated into the host cell genome will comprise an inactivated 5' LTR.

The vectors provided herein typically comprise a gene that encodes a protein (or other molecule, such as siRNA) that is desirably expressed in one or more target cells. In a viral vector, the gene of interest is preferably located between the 5' LTR and 3' LTR sequences. Further, the gene of interest is preferably in a functional relationship with other genetic elements, for example, transcription regulatory sequences such as promoters and/or enhancers, to regulate expression of the gene of interest in a particular manner once the gene is incorporated into the target cell. In certain embodiments, the useful transcriptional regulatory sequences are those that are highly regulated with respect to activity, both temporally and spatially.

In certain embodiments, one or more additional genes may be incorporated as a safety measure, mainly to allow for the selective killing of transfected target cells within a heterogeneous population, such as within a human patient. In one exemplary embodiment, the selected gene is a thymidine kinase gene (TK), the expression of which renders a target cell susceptible to the action of the drug gancyclovir. In a further embodiment, the suicide gene is a caspase 9 suicide gene activated by a dimerizing drug (see, e.g., Tey et al., Biology of Blood and Marrow Transplantation 13:913-924, 2007).

In certain embodiments, a gene encoding a marker protein may be placed before or after the primary gene in a viral or non-viral vector to allow for identification and/or selection of cells that are expressing the desired protein. Certain embodiments incorporate a fluorescent marker protein, such as green fluorescent protein (GFP) or red fluorescent protein (RFP), along with the primary gene of interest. If one or more additional reporter genes are included, IRES sequences or 2A elements may also be included, separating the primary gene of interest from a reporter gene and/or any other gene of interest.

Certain embodiments may employ genes that encode one or more selectable markers. Examples include selectable markers that are effective in a eukaryotic cell or a prokaryotic cell, such as a gene for a drug resistance that encodes a factor necessary for the survival or growth of transformed host cells grown in a selective culture medium. Exemplary selection genes encode proteins that confer resistance to antibiotics or other toxins, e.g., G418, hygromycin B, puromycin, zeocin, ouabain, blasticidin, ampicillin, neomycin, methotrexate, or tetracycline, complement auxotrophic deficiencies, or supply may be present on a separate plasmid and introduced by co-transfection with the viral vector. In one embodiment, the gene encodes for a mutant dihydrofolate reductase (DHFR) that confers methotrexate resistance. Certain other embodiments may employ genes that encode one or cell surface receptors that can be used for tagging and detection or purification of transfected cells (*e*.*g*., low-affinity nerve growth factor receptor (LNGFR) or other such receptors useful as transduction tag systems. See *e*.*g*., Lauer et al., Cancer Gene Ther. 2000 Mar;7(3):430-7.

Certain viral vectors such as retroviral vectors employ one or more heterologous promoters, enhancers, or both. In certain embodiments, the U3 sequence from a retroviral or lentiviral 5' LTR may be replaced with a promoter or enhancer sequence in the viral construct. Certain embodiments employ an "internal" promoter/enhancer that is located between the 5' LTR and 3' LTR sequences of the viral vector, and is operably linked to the gene of interest.

A "functional relationship" and "operably linked" mean, without limitation, that the gene is in the correct location and orientation with respect to the promoter and/or enhancer, such that expression of the gene will be affected when the promoter and/or enhancer is contacted with the appropriate regulatory molecules. Any enhancer/promoter combination may be used that either regulates (*e*.*g*., increases, decreases) expression of the viral RNA genome in the packaging cell line, regulates expression of the selected gene of interest in an infected target cell, or both.

A promoter is an expression control element formed by a DNA sequence that permits polymerase binding and transcription to occur. Promoters are untranslated sequences that are located upstream (5') of the start codon of a selected gene of interest (typically within about 100 to 1000 bp) and control the transcription and translation of the coding polynucleotide sequence to which they are operably linked. Promoters may be inducible or constitutive. Inducible promoters initiate increased levels of transcription from DNA under their control in response to some change in culture conditions, such as a change in temperature. Promoters may be unidirectional or bidirectional. Bidirectional promoters can be used to co-express two genes, e.g., a gene of interest and a selection marker. Alternatively, a bidirectional promoter configuration comprising two promoters, each controlling expression of a different gene, in opposite orientation in the same vector may be utilized.

A variety of promoters are known in the art, as are methods for operably linking the promoter to the polynucleotide coding sequence. Both native promoter sequences and many heterologous promoters may be used to direct expression of the selected gene of interest. Certain embodiments employ heterologous promoters, because they generally permit greater transcription and higher yields of the desired protein as compared to the native promoter.

Certain embodiments may employ heterologous viral promoters. Examples of such promoters include those obtained from the genomes of viruses such as polyoma virus, fowlpox virus, adenovirus, bovine papilloma virus, avian sarcoma virus, cytomegalovirus, a retrovirus, hepatitis-B virus and Simian Virus 40 (SV40). Certain embodiments may employ heterologous mammalian promoter, such as the actin promoter, an immunoglobulin promoter, a heat-shock promoter, or a promoter that is associated with the native sequence of the gene of interest. Typically, the promoter is compatible with the target cell, such as an activated B-lymphocyte, a plasma B cell, a memory B cell or other lymphocyte target cell.

Certain embodiments may employ one or more of the RNA polymerase II and III promoters. A suitable selection of RNA polymerase III promoters can be found, for example, in Paule and White. Nucleic Acids Research., Vol. 28, pp 1283-1298, 2000, which is incorporated by reference in its entirety. RNA polymerase II and III promoters also include any synthetic or engineered DNA fragments that can direct RNA polymerase II or III, respectively, to transcribe its downstream RNA coding sequences. Further, the RNA polymerase II or III (Pol II or III) promoter or promoters used as part of the viral vector can be inducible. Any suitable inducible Pol II or III promoter can be used with the methods described herein. Exemplary Pol II or III promoters include the tetracycline responsive promoters provided in Ohkawa and Taira, Human Gene Therapy, Vol. 11, pp 577-585, 2000; and Meissner et al., Nucleic Acids Research, Vol. 29, pp 1672-1682, 2001, each of which is incorporated by reference in its entirety.

Non-limiting examples of constitutive promoters that may be used include the promoter for ubiquitin, the CMV promoter (see, e.g., Karasuyama et al., J. Exp. Med. 169:13, 1989), the β-actin (see, e.g., Gunning et al., PNAS USA 84:4831 -4835, 1987), the elongation factor-1 alpha (EF-1 alpha) promoter, the CAG promoter, and the pgk promoter (see, e.g., Adra et al., Gene 60:65-74, 1987); Singer-Sam et al., Gene 32:409-417, 1984; and Dobson et al., Nucleic Acids Res. 10:2635-2637, 1982, each of which is incorporated by reference). Non-limiting examples of tissue specific promoters include the Ick promoter (see, *e*.*g*., Garvin et al., Mol. Cell Biol. 8:3058-3064, 1988; and Takadera et al., Mol. Cell Biol. 9:2173-2180, 1989), the myogenin promoter (Yee et al., Genes and Development 7:1277-1289. 1993), and the thyl promoter (see, *e*.*g*., Gundersen et al., Gene 1 13:207-214, 1992).

Additional examples of promoters include the ubiquitin-C promoter, the human µ heavy chain promoter or the Ig heavy chain promoter (*e*.*g*., MH), and the human κ light chain promoter or the Ig light chain promoter (*e*.*g*., EEK), which are functional in B-lymphocytes. The MH promoter contains the human µ heavy chain promoter preceded by the iEµ enhancer flanked by matrix association regions, and the EEK promoter contains the κ light chain promoter preceded an intronic enhancer (iEκ), a matrix associated region, and a 3' enhancer (3Eκ) (see, *e*.*g*., Luo et al., Blood. 1 13:1422-1431 , 2009, and U.S. Patent Application Publication No. 2010/0203630). Accordingly, certain embodiments may employ one or more of these promoter or enhancer elements.

In one embodiment, one promoter drives expression of a selectable marker and a second promoter drives expression of the gene of interest. For example, in one embodiment, the EF-1 alpha promoter drives the production of a selection marker (*e*.*g*., DHFR) and a miniature CAG promoter (see, *e*.*g*., Fan et al. Human Gene Therapy 10:2273-2285, 1999) drives expression of the gene of interest (*e*.*g*., IDUA).

As noted above, certain embodiments employ enhancer elements, such as an internal enhancer, to increase expression of the gene of interest. Enhancers are cis-acting elements of DNA, usually about 10 to 300 bp in length, that act on a promoter to increase its transcription. Enhancer sequences may be derived from mammalian genes (*e*.*g*., globin, elastase, albumin, α-fetoprotein, insulin), such as the ιεµ enhancer, the ιεκ intronic enhancer, and the 3' εκ enhancer. Also included are enhancers from a eukaryotic virus, including the SV40 enhancer on the late side of the replication origin (bp 100-270), the cytomegalovirus early promoter enhancer, the polyoma enhancer on the late side of the replication origin, and adenovirus enhancers. Enhancers may be spliced into the vector at a position 5' or 3' to the antigen-specific polynucleotide sequence, but are preferably located at a site 5' from the promoter. Persons of skill in the art will select the appropriate enhancer based on the desired expression pattern.

In certain embodiments, promoters are selected to allow for inducible expression of the gene. A number of systems for inducible expression are known in the art, including the tetracycline responsive system and the lac operator-repressor system. It is also contemplated that a combination of promoters may be used to obtain the desired expression of the gene of interest. The skilled artisan will be able to select a promoter based on the desired expression pattern of the gene in the organism and/or the target cell of interest.

Certain viral vectors contain cis-acting packaging sequences to promote incorporation of the genomic viral RNA into the viral particle. Examples include psi-sequences. Such cis-acting sequences are known in the art. In certain embodiments, the viral vectors described herein may express two or more genes, which may be accomplished, for example, by incorporating an internal promoter that is operably linked to each separate gene beyond the first gene, by incorporating an element that facilitates co-expression such as an internal ribosomal entry sequence (IRES) element (U.S. Pat. No. 4,937,190, incorporated by reference) or a 2A element, or both. Merely by way of illustration, IRES or 2A elements may be used when a single vector comprises sequences encoding each chain of an immunoglobulin molecule with a desired specificity. For instance, the first coding region (encoding either the heavy or light chain) may be located immediately downstream from the promoter, and the second coding region (encoding the other chain) may be located downstream from the first coding region, with an IRES or 2A element located between the first and second coding regions, preferably immediately preceding the second coding region. In other embodiments, an IRES or 2A element is used to co-express an unrelated gene, such as a reporter gene, a selectable marker, or a gene that enhances immune function. Examples of IRES sequences that can be used include, without limitation, the IRES elements of encephalomyelitis virus (EMCV), foot-and- mouth disease virus (FMDV), Theiler's murine encephalomyelitis virus (TMEV), human rhinovirus (HRV), coxsackievirus (CSV), poliovirus (POLIO), Hepatitis A virus (HAV), Hepatitis C virus (HCV), and Pestiviruses (*e*.*g*., hog cholera virus (HOCV) and bovine viral diarrhea virus (BVDV)) (see, *e*.*g*., Le et al., Virus Genes 12:135-147, 1996; and Le et al., Nuc. Acids Res. 25:362-369, 1997, each of which is incorporated by reference in their entirety). One example of a 2A element includes the F2A sequence from foot-and-mouth disease virus.

In certain embodiments, the vectors provided herein also contain additional genetic elements to achieve a desired result. For example, certain viral vectors may include a signal that facilitates nuclear entry of the viral genome in the target cell, such as an HIV-1 flap signal. As a further example, certain viral vectors may include elements that facilitate the characterization of the provirus integration site in the target cell, such as a tRNA amber suppressor sequence. Certain viral vectors may contain one or more genetic elements designed to enhance expression of the gene of interest. For example, a woodchuck hepatitis virus responsive element (WRE) may be placed into the construct (see, *e*.*g*., Zufferey et al., J. Virol. 74:3668-3681, 1999; and Deglon et al., Hum. Gene Ther. 11:179-190, 2000, each of which is incorporated by reference in its entirety). As another example, a chicken β-globin insulator may also be included in the construct. This element has been shown to reduce the chance of silencing the integrated DNA in the target cell due to methylation and heterochromatinization effects. In addition, the insulator may shield the internal enhancer, promoter and exogenous gene from positive or negative positional effects from surrounding DNA at the integration site on the chromosome. Certain embodiments employ each of these genetic elements. In another embodiment, the viral vectors provided herein may also contain a Ubiquitous Chromatin Opening Element (UCOE) to increase expression (see *e*.*g*., Zhang F, et al., Molecular Therapy: The journal of the American Society of Gene Therapy 2010 Sep;18(9):1640-9.)

In certain embodiments, the viral vectors (*e*.*g*., retroviral, lentiviral) provided herein are "pseudo-typed" with one or more selected viral glycoproteins or envelope proteins, mainly to target selected cell types. Pseudo-typing refers to generally to the incorporation of one or more heterologous viral glycoproteins onto the cell-surface virus particle, often allowing the virus particle to infect a selected cell that differs from its normal target cells. A "heterologous" element is derived from a virus other than the virus from which the RNA genome of the viral vector is derived. Typically, the glycoprotein-coding regions of the viral vector have been genetically altered such as by deletion to prevent expression of its own glycoprotein. Merely by way of illustration, the envelope glycoproteins gp41 and/or gp120 from an HIV-derived lentiviral vector are typically deleted prior to pseudo-typing with a heterologous viral glycoprotein.

In certain embodiments, the viral vector is pseudo-typed with a heterologous viral glycoprotein that targets B lymphocytes. In certain embodiments, the viral glycoprotein allows selective infection or transduction of resting or quiescent B lymphocytes. In certain embodiments, the viral glycoprotein allows selective infection of B lymphocyte plasma cells, plasmablasts, and activated B cells. In certain embodiments, the viral glycoprotein allows infection or transduction of quiescent B lymphocytes, plasmablasts, plasma cells, and activated B cells. In certain embodiments, viral glycoprotein allows infection of B cell chronic lymphocyte leukemia cells. In one embodiment, the viral vector is pseudo-typed with VSV-G. In another embodiment, the heterologous viral glycoprotein is derived from the glycoprotein of the measles virus, such as the Edmonton measles virus. Certain embodiments pseudo-type the measles virus glycoproteins hemagglutinin (H), fusion protein (F), or both (see, *e*.*g*., Frecha et al., Blood. 1 12:4843-52, 2008; and Frecha et al., Blood. 1 14:3173-80, 2009, each of which is incorporated by reference in its entirety). In one embodiment, the viral vector is pseudo-typed with gibbon ape leukemia virus (GALV). In further embodiments, the viral vector comprises an embedded antibody binding domain, such as one or more variable regions (*e*.*g*., heavy and light chain variable regions) which serves to target the vector to a particular cell type.

Generation of viral vectors can be accomplished using any suitable genetic engineering techniques known in the art, including, without limitation, the standard techniques of restriction endonuclease digestion, ligation, transformation, plasmid purification, PCR amplification, and DNA sequencing, for example as described in Sambrook et al. (Molecular Cloning: A Laboratory Manual. Cold Spring Harbor Laboratory Press, N.Y. (1989)), Coffin et al. (Retroviruses. Cold Spring Harbor Laboratory Press, N.Y. (1997)) and "RNA Viruses: A Practical Approach" (Alan J. Cann, Ed., Oxford University Press, (2000)).

Any variety of methods known in the art may be used to produce suitable retroviral particles whose genome comprises an RNA copy of the viral vector. As one method, the viral vector may be introduced into a packaging cell line that packages the viral genomic RNA based on the viral vector into viral particles with a desired target cell specificity. The packaging cell line typically provides in trans the viral proteins that are required for packaging the viral genomic RNA into viral particles and infecting the target cell, including the structural gag proteins, the enzymatic pol proteins, and the envelope glycoproteins.

In certain embodiments, the packaging cell line stably expresses certain necessary or desired viral proteins (*e*.*g*., gag, pol) (see, *e*.*g*., U.S. Pat. No. 6,218,181, herein incorporated by reference). In certain embodiments, the packaging cell line is transiently transfected with plasmids that encode certain of the necessary or desired viral proteins (*e*.*g*., gag, pol, glycoprotein), including the measles virus glycoprotein sequences described herein. In one exemplary embodiment, the packaging cell line stably expresses the gag and pol sequences, and the cell line is then transfected with a plasmid encoding the viral vector and a plasmid encoding the glycoprotein. Following introduction of the desired plasmids, viral particles are collected and processed accordingly, such as by ultracentrifugation to achieve a concentrated stock of viral particles. Exemplary packaging cell lines include 293 (ATCC CCL X), HeLa (ATCC CCL 2), D17 (ATCC CCL 183), MDCK (ATCC CCL 34), BHK (ATCC CCL-10) and Cf2Th (ATCC CRL 1430) cell lines.

### Gene of Interest

As used herein "gene of interest" or "gene" or "nucleic acid of interest" refers to a transgene to be expressed in the target transfected cell. While the term "gene" may be used, this is not to imply that this is a gene as found in genomic DNA and is used interchangeably with the term "nucleic acid". Generally, the nucleic acid of interest provides suitable nucleic acid for encoding the protein of interest and may comprise cDNA or DNA and may or may not include introns but generally does not include introns. As noted elsewhere, the nucleic acid of interest is operably linked to expression control sequences to effectively express the protein of interest in the target cell. In certain embodiments, the vectors described herein may comprise one or more genes of interest, and may include 2, 3, 4, or 5 or more genes of interest, such as for example, the heavy and light chains of an immunoglobulin that may be organized using an internal promoter as described herein.

The recitation "polynucleotide" or "nucleic acid" as used herein designates mRNA, RNA, cRNA, cDNA or DNA. The term typically refers to polymeric form of nucleotides of at least 10 bases in length, either ribonucleotides or deoxynucleotides or a modified form of either type of nucleotide. The term includes single and double stranded forms of DNA and RNA. The nucleic acid or gene of interest may be any nucleic acid encoding a protein of interest.

A protein of interest for use as described herein comprises any protein providing an activity desired. In this regard, a protein of interest includes, but is not limited to, an antibody or antigen-binding fragment thereof, a cell surface receptor, a secreted protein such as a cytokine (lymphokines, interleukins, interferons, or chemokines), other secreted signaling molecules such as TGF-beta and fibroblast growth factor, an antigenic fragment of a protein, a DNA-encoded small molecule (see *e*.*g*., Nature Chemical Biology 5, 647 - 654 (2009)), an enzyme, a clotting factor, and an adhesion molecule. In one embodiment, the nucleic acid encodes an antibody or antigen-binding fragment thereof. Exemplary antigen binding fragments include domain antibodies, sFv, scFv, Fab, Fab', F(ab')2, and Fv. In one embodiment, the nucleic acid encodes the protein of interest as a fusion protein comprising a cleavable linker. For example, an antibody heavy chain and a light chain can be expressed with a self-cleavable linker peptide, e.g., F2A.

In one embodiment, the antibody encoded by the nucleic acid comprises at least the antigen binding domain of the HIV neutralizing antibody, b12 (see, *e*.*g*., J Virol 2003, 77:5863- 5876; J Virol. 1994 Aug; 68(8):4821 -8; Proc Natl Acad Sci U S A. 1992, 89:9339-9343; exemplary sequences are provided in GenBank Accession Nos. for the b12 light chain (AAB26306.1 GI 299737) and heavy chain (AAB26315.1 GI 299746)). In a further embodiment, the antibody encoded by the nucleic acid of interest comprises Fuzeon(TM) (T-20 / enfuvirtide / pentafuside / DP-178). DP-178 is an amino acid sequence from gp41 on HIV and interferes with HIV's ability to fuse with its target cell. Fuzeon may be produced synthetically using methods known to the skilled person (see *e*.*g*., 2001 J. Virol. 75:3038-3042; It should be noted that it is highly unlikely that the methods described in this paper resulted in secretion of a therapeutic dose of the DP-178 peptide).

In one particular embodiment, the nucleic acid of interest encodes an immunologically active protein. In certain embodiments, a nucleic acid of interest encodes a protein, or a biologically active fragment thereof (*e*.*g*., an antigenic fragment), that induces an immune vaccine-like reaction through the presentation of the protein on the surface of a B cell, T cell or other immune cell. In certain embodiments, the protein of interest influences the regulation of B cells, for example but not limited to promoting cell division, promoting differentiation into different B lineages, inactivating or killing cells, or regulates production or activity of other introduced DNA elements. Interleukins are known to the skilled person and to date include IL-1 , IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21 , IL-22, IL-23, IL-24, IL- 25, IL-26, IL-27, secreted form of the p28 subunit of IL27, IL-28, IL-29, IL-30, IL-31, IL-32, IL-33, IL-34, and IL-35. Interferons include IFN-γ, IFN-α, IFN-β and IFN-o. The chemokines contemplated for use herein include the C type chemokines XCL1 and XCL2, C-C type chemokines (to date including CCL1 - CCL28) and CXC type chemokines (to date including CXCL1 -CXCL17). Also contemplated as a gene of interest are members of the TNF superfamily (*e*.*g*., TNF-a, 4-1 BB ligand, B cell activating factor, FAS ligand, Lymphotoxin, OX40L RANKL, and TRAIL).

In certain embodiments, the protein of interest induces immunological tolerance. In this regard, the protein of interest may comprise an IgG-antigen fusion protein (see *e*.*g*., Cellular Immunology 235(1), 2005, 12-20). In certain embodiments, expression of a protein of interest may be accompanied by stimulation of the cells with factors such as TGF-β, IL-10 and LPS. In certain embodiments, factors such as IL-10 or transcription factors that induce tolerance are expressed with the cultured B cells.

In a further embodiment, the gene(s) of interest encodes one or more factors that promote differentiation of the B cell into an antibody secreting cell and/or one or more factors that promote the longevity of the antibody producing cell. Such factors include, for example, Blimp-1, Xbp1, IRF4, Zbtb20, TRF4, anti-apoptotic factors like Bcl-xl, Bcl-2, Mcl-1, or Bcl5, constitutively active mutants of the CD40 receptor. Further genes of interest encode factors which promote the expression of downstream signaling molecules such as TNF receptor- associated factors (TRAFs). In this regard, cell activation, cell survival, and antiapoptotic functions of the TNF receptor superfamily are mostly mediated by TRAF 1 -6 (see *e*.*g*., R.H. Arch, et al., Genes Dev. 12 (1998), pp. 2821-2830). Downstream effectors of TRAF signaling include transcription factors in the NF- KB and AP-1 family which can turn on genes involved in various aspects of cellular and immune functions. Further, the activation of NF-κβ and AP-1 has been shown to provide cells protection from apoptosis via the transcription of anti-apoptotic genes. In an additional embodiment the encoded factor, such as IL-10, IL-35, TGF-beta or an Fc-fusion protein, is associated with induction of immune tolerance.

In an additional embodiment, the nucleic acid(s) of interest encodes one or more Epstein Barr virus (EBV)-derived proteins. EBV-derived proteins include but are not limited to, EBNA-1, EBNA-2, EBNA-3, LMP-1, LMP-2, EBER, EBV-EA, EBV-MA, EBV-VCA and EBV-AN. In one particular embodiment, the nucleic acid of interest encodes an antibody or an antigen-binding fragment thereof. In this regard, the antibody may be a natural antibody or a custom, recombinantly engineered antibody. Fusion proteins comprising an antibody or portion thereof are specifically contemplated to be encoded by the vectors described herein.

In one embodiment, an antibody or fragment thereof according to the present disclosure has an amino acid sequence of an anti-HIV antibody, such as the m36 anti-HIV antibody (see *e*.*g*., Proc Natl Acad Sci U S A. 2008 Nov 4;105(44):17121 -6), or an amino acid molecule having at least 60%, 80%, 85%, 90%, 95%, 96%, 97%, 98% or 99% sequence identity with an amino acid sequence of an anti-HIV antibody, such as m36. In particular, fusion proteins comprising m36, or derivatives thereof, are specifically contemplated, such as m36L2CD4Fc (see *e*.*g*., Antiviral Research volume 88, Issue 1, October 2010, Pages 107-1 15). In one embodiment, the anti-HIV antibody is the broadly neutralizing monoclonal antibody VRC01 (see, *e*.*g*., Wu et al., Science, 2010, 329(5993):856861 and Li et al., J Virol, 2011, 85(17):8954-8967).

In a further embodiment, the antibody encoded by the transgene of the disclosure binds to an autoantigen. In certain embodiments, the autoantigen in this regard is associated with the development of multiple sclerosis or Type 1 diabetes, including but not limited to MBP, alphaB-crystallin, S100beta, proteolipid protein (PLP), HSP105, epithelial isoform of bullous pemphigoid (BP) antigen 1 (BPAG1-e), lipids, and myelin oligodendrocyte glycoprotein (MOG)-alpha and MOG-beta isoforms or any of a variety of islet cell autoantigens (*e*.*g*., sialoglycolipid, glutamate decarboxylase, insulin, insulin receptor, 38 kD, bovine serum albumin, glucose transporter, hsp 65, carboxypeptidase H, 52 kD, ICA 12/ICA512, 150 kD, and RIN polar). Antibodies to these autoantigens are known in the art and may be sequenced and made recombinantly using routine techniques (see *e*.*g*., J. Clin. Invest. 107(5): 555-564 (2001)).

In a further embodiment, the antibody binds to a cancer-associated antigen. Cancer-associated antigens may be derived from a variety of tumor proteins. Illustrative tumor proteins useful in the present disclosure include, but are not limited to any one or more of, p53, MAGE-A1 , MAGE-A2, MAGE-A3, MAGE-A4, MAGE-A6, MAGE-A10, MAGE-A12, BAGE, DAM-6, -10, GAGE-1 , -2, -8, GAGE-3, -4, -5, -6, -7B, NA88-A, NY-ESO-1 , MART-1 , MC1 R, Gp100, PSA, PSM, Tyrosinase, TRP-1 , TRP-2, ART-4, CAMEL, CEA, Cyp-B, Her2/neu (*e*.*g*., the antibody may be derived from the Her2-specific mAb, Herceptin(R)), hTERT, hTRT, iCE, MUC1 , MUC2, PRAME, P15, RU1 , RU2, SART-1 , SART-3, WT1 , AFP, β-catenin/m, Caspase-8/m, CEA, CDK-4/m, ELF2M, GnT-V, G250, HSP70-2M, HST-2, KIAA0205, MUM-1 , MUM-2, MUM-3, Myosin/m, RAGE, SART-2, TRP-2/INT2, 707-AP, Annexin II, CDC27/m, TPI/mbcr-abl, ETV6/AML, LDLR/FUT, Pml/RARa, and TEL/AML1 . These and other tumor proteins are known to the skilled artisan.

In further embodiments, the nucleic acid of interest encodes a peptide or other binding domain with a particular functional attribute, such as, but not limited to, an inhibitory activity, ability to induce cell death in cancer cells, or ability to slow or inhibit cancer cell proliferation. In this regard, in one embodiment, a peptide or binding domain encoded by the nucleic acid of interest may bind any of the target proteins described herein, such as a cancer-associated antigen as described above, CD4, HIV gp120 or other viral protein, ICAM-3, DC-SIGN (see *e*.*g*., U.S. patent 7,301,010). In certain embodiments, the peptides may be derived from pathogenic and nonpathogenic bacteria and green plants. Illustrative peptides are disclosed in U.S. patents 7084105, 7301010, 7338766, 7381701, 7491394, 7511117, 7556810. In one embodiment, the nucleic acid of interest encodes azurin-p28 (NSC745104) a peptide inhibitor of p53 ubiquitination (see *e*.*g*., Cancer Chemother Pharmacol 2010, DOI 10.1007/S00280-010-1518-3; U.S. Patent 7,084,105). In a further embodiment, the nucleic acid of interest encodes a factor known as Ghrelin, which induces appetite and can be used to treat cancer patients (see *e*.*g*., Obes Facts. 2010 3:285-92; FASEB J. 18 (3): 439-56). In another embodiment, the nucleic acid of interest encodes a binding peptide that binds to and inhibits angiopoietin 1 and 2 (see, *e*.*g*., AMG386, an Fc fragment of an antibody (peptibody) used to treat cancer; In certain embodiments, tumor antigens may be identified directly from an individual with cancer. In this regard, screens can be carried out using a variety of known technologies. For example, in one embodiment, a tumor biopsy is taken from a patient, RNA is isolated from the tumor cells and screened using a gene chip (for example, from Affymetrix, Santa Clara, CA) and a tumor antigen is identified. Once the tumor target antigen is identified, it may then be cloned, expressed and purified using techniques known in the art.

In one particular embodiment, the nucleic acid of interest encodes an enzyme. In one embodiment, the nucleic acid of interest encodes an enzyme to treat a lysosomal storage disorder. In one embodiment, the nucleic acid of interest encodes iduronidase (IDUA) for treatment or prevention of mucopolysaccharidosis type I (MPS I). In one embodiment, the nucleic acid of interest encodes idursulfase for treatment or prevention of mucopolysaccharidosis type II (MPS II). In one embodiment, the nucleic acid of interest encodes galsulfase for treatment or prevention of mucopolysaccharidosis type VI (MPS VI). In one embodiment, the nucleic acid of interest encodes elosulfase alfa for treatment or prevention of mucopolysaccharidosis type IVA (MPS IVA). In one embodiment, the nucleic acid of interest encodes agalsidase beta for treatment or prevention of Fabry's disease. In one embodiment, the nucleic acid of interest encodes agalsidase alpha for treatment or prevention of Fabry's disease. In one embodiment, the nucleic acid of interest encodes alpha-1-anti-trypsin for treatment or prevention of Alpha-1-anti-trypsin deficiency. In one embodiment, the nucleic acid of interest encodes alpha-N-acetylglucosaminidase for treatment or prevention of mucopolysaccharidosis type IIIB (MPS IIIB). In another embodiment, the nucleic acid of interest encodes factor VII for treatment or prevention of hemophilia. In one embodiment, the nucleic acid of interest encodes lecithin-cholesterol acyltransferase (LCAT) useful for treatment or prevention of, *e*.*g*., LCAT deficiency and atherosclerosis. In another embodiment, the nucleic acid of interest encodes Apolipoprotein A-1 Milano (ApoA-1 Milano) for treatment or prevention of cardiovascular diseases and disorders, such as, *e*.*g*., atherosclerosis. In one embodiment, the nucleic acid of interest encodes lipoprotein lipase (LPL) for treatment or prevention of LPL deficiency. In another embodiment, the nucleic acid of interest encodes a broadly neutralizing antibody (bNAb), or a fusion protein thereof, that binds to and neutralizes multiple HIV-1 strains (*e*.*g*., b12). In yet another embodiment, the nucleic acid of interest encodes phenylalanine hydroxylase for treatment or prevention of phenyketonuria (PKU).

An "antibody", as used herein, includes both polyclonal and monoclonal antibodies; primatized (*e*.*g*., humanized); murine; mouse-human; mouse-primate; and chimeric; and may be an intact molecule, a fragment thereof (such as scFv, Fv, Fd, Fab, Fab' and F(ab)'2 fragments), or multimers or aggregates of intact molecules and/or fragments; and may occur in nature or be produced, *e*.*g*., by immunization, synthesis or genetic engineering; an "antibody fragment," as used herein, refers to fragments, derived from or related to an antibody, which bind antigen and which in some embodiments may be derivatized to exhibit structural features that facilitate clearance and uptake, *e*.*g*., by the incorporation of galactose residues. This includes, *e*.*g*., F(ab), F(ab)'2, scFv, light chain variable region (VL), heavy chain variable region (VH), and combinations thereof. Sources include antibody gene sequences from various species (which can be formatted as antibodies, sFvs, scFvs or Fabs, such as in a phage library), including human, camelid (from camels, dromedaries, or llamas; Hamers-Casterman et al. (1993) Nature, 363:446 and Nguyen et al. (1998) J. Mol. Biol., 275:413), shark (Roux et al. (1998) Proc. Nat'l. Acad. Sci. (USA) 95:1 1804), fish (Nguyen et al. (2002) Immunogenetics, 54:39), rodent, avian, ovine, sequences that encode random peptide libraries or sequences that encode an engineered diversity of amino acids in loop regions of alternative non-antibody scaffolds, such as fibrinogen domains (see, *e*.*g*., Weisel et al. (1985) Science 230:1388), Kunitz domains (see, *e*.*g*., US Patent No. 6,423,498), lipocalin domains (see, *e*.*g*., WO 2006/095164), V-like domains (see, *e*.*g*., US Patent Application Publication No. 2007/0065431 ), C-type lectin domains (Zelensky and Gready (2005) FEBS J. 272:6179), mAb<2> or Fcab(TM) (see, *e*.*g*., PCT Patent Application Publication Nos. WO 2007/098934; WO 2006/072620), or the like.

Terms understood by those in the art as referring to antibody technology are each given the meaning acquired in the art, unless expressly defined herein. For example, the terms "VL" and "VH" refer to the variable binding region derived from an antibody light and heavy chain, respectively. The variable binding regions are made up of discrete, well-defined sub-regions known as "complementarity determining regions" (CDRs) and "framework regions" (FRs). The terms "CL" and "CH" refer to an "immunoglobulin constant region," *i*.*e*., a constant region derived from an antibody light or heavy chain, respectively, with the latter region understood to be further divisible into Cm, CH2, CH3 and CH4 constant region domains, depending on the antibody isotype (IgA, IgD, IgE, IgG, IgM) from which the region was derived. A portion of the constant region domains makes up the Fc region (the "fragment crystallizable" region), which contains domains responsible for the effector functions of an immunoglobulin, such as ADCC (antibody-dependent cell-mediated cytotoxicity), CDC (complement-dependent cytotoxicity) and complement fixation, binding to Fc receptors, greater half-life *in vivo* relative to a polypeptide lacking an Fc region, protein A binding, and perhaps even placental transfer (see Capon et al. (1989) Nature, 337:525). Further, a polypeptide containing an Fc region allows for dimerization or multimerization of the polypeptide.

The domain structure of immunoglobulins is amenable to engineering, in that the antigen binding domains and the domains conferring effector functions may be exchanged between immunoglobulin classes and subclasses. For example, amino acid changes (*e*.*g*., deletions, insertions, substitutions) may alter post-translational processes of the immunoglobulin, such as changing the number or position of glycosylation and/or fucosylation sites. Methods for enhancing ADCC via glycosylation are known in the art and contemplated for use herein. For example, enzymes that enhance glycosylation may be co-expressed with the antibody. In one embodiment, MGAT3 is overexpressed in cells producing the antibody to enhance glycosylation of the antibody and its ADCC function. In one embodiment, inhibition of Fut8 via, e.g., siRNA, enhances glycosylation of the antibody and ADCC.

Immunoglobulin structure and function are reviewed, for example, in Harlow et al., Eds., Antibodies: A Laboratory Manual, Chapter 14 (Cold Spring Harbor Laboratory, Cold Spring Harbor, 1988). An extensive introduction as well as detailed information about all aspects of recombinant antibody technology can be found in the textbook Recombinant Antibodies (John Wiley & Sons, NY, 1999). A comprehensive collection of detailed antibody engineering lab Protocols can be found in R. Kontermann and S. Dubel, Eds., The Antibody Engineering Lab Manual (Springer Verlag, Heidelberg/New York, 2000). Further related protocols are also available in Current Protocols in Immunology (August 2009,) published by John Wiley & Sons, Inc., Boston, MA. Methods for production of enzymes and protein engineering (*e*.*g*., IDUA) are also known in the art and contemplated for use herein.

Thus, this disclosure provides polynucleotides (isolated or purified or pure polynucleotides) encoding the proteins of interest of this disclosure for genetically modifying B cells, vectors (including cloning vectors and expression vectors) comprising such polynucleotides, and cells (*e*.*g*., host cells) transformed or transfected with a polynucleotide or vector according to this disclosure. In certain embodiments, a polynucleotide (DNA or RNA) encoding a protein of interest of this disclosure is contemplated. Expression cassettes encoding proteins of interest are also contemplated herein.

The present disclosure also relates to vectors that include a polynucleotide of this disclosure and, in particular, to recombinant expression constructs. In one embodiment, this disclosure contemplates a vector comprising a polynucleotide encoding a protein of this disclosure, along with other polynucleotide sequences that cause or facilitate transcription, translation, and processing of such a protein-encoding sequences. Appropriate cloning and expression vectors for use with prokaryotic and eukaryotic hosts are described, for example, in Sambrook et ai, Molecular Cloning: A Laboratory Manual, Second Edition, Cold Spring Harbor, NY, (1989). Exemplary cloning/expression vectors include cloning vectors, shuttle vectors, and expression constructs, that may be based on plasmids, phagemids, phasmids, cosmids, viruses, artificial chromosomes, or any nucleic acid vehicle known in the art suitable for amplification, transfer, and/or expression of a polynucleotide contained therein.

As used herein, unless as otherwise described with regard to viral vectors, "vector" means a nucleic acid molecule capable of transporting another nucleic acid to which it has been linked. Exemplary vectors include plasmids, minicircles, transposons, yeast artificial chromosomes, self-replicating RNAs, and viral genomes. Certain vectors can autonomously replicate in a host cell, while other vectors can be integrated into the genome of a host cell and thereby are replicated with the host genome. In addition, certain vectors are referred to herein as "recombinant expression vectors" (or simply, "expression vectors"), which contain nucleic acid sequences that are operatively linked to an expression control sequence and, therefore, are capable of directing the expression of those sequences. In certain embodiments, expression constructs are derived from plasmid vectors. Illustrative constructs include modified pNASS vector (Clontech, Palo Alto, CA), which has nucleic acid sequences encoding an ampicillin resistance gene, a polyadenylation signal and a T7 promoter site; pDEF38 and pNEF38 (CMC ICOS Biologies, Inc.), which have a CHEF1 promoter; and pD18 (Lonza), which has a CMV promoter. Other suitable mammalian expression vectors are well known (see, e.g., Ausubel et al., 1995; Sambrook et al., supra; see also, *e*.*g*., catalogs from Invitrogen, San Diego, CA; Novagen, Madison, Wl; Pharmacia, Piscataway, NJ). Useful constructs may be prepared that include a dihydrofolate reductase (DHFR)-encoding sequence under suitable regulatory control, for promoting enhanced production levels of the fusion proteins, which levels result from gene amplification following application of an appropriate selection agent (*e*.*g*., methotrexate).

Generally, recombinant expression vectors will include origins of replication and selectable markers permitting transformation of the host cell, and a promoter derived from a highly-expressed gene to direct transcription of a downstream structural sequence, as described above. A vector in operable linkage with a polynucleotide according to this disclosure yields a cloning or expression construct. Exemplary cloning/expression constructs contain at least one expression control element, *e*.*g*., a promoter, operably linked to a polynucleotide of this disclosure. Additional expression control elements, such as enhancers, factor-specific binding sites, terminators, and ribosome binding sites are also contemplated in the vectors and cloning/expression constructs according to this disclosure. The heterologous structural sequence of the polynucleotide according to this disclosure is assembled in appropriate phase with translation initiation and termination sequences. Thus, for example, encoding nucleic acids as provided herein may be included in any one of a variety of expression vector constructs (*e*.*g*., minicircles) as a recombinant expression construct for expressing such a protein in a host cell.

The appropriate DNA sequence(s) may be inserted into a vector, for example, by a variety of procedures. In general, a DNA sequence is inserted into an appropriate restriction endonuclease cleavage site(s) by procedures known in the art. Standard techniques for cloning, DNA isolation, amplification and purification, for enzymatic reactions involving DNA ligase, DNA polymerase, restriction endonucleases and the like, and various separation techniques are contemplated. A number of standard techniques are described, for example, in Ausubel et al. (Current Protocols in Molecular Biology, Greene Publ. Assoc. Inc. & John Wiley & Sons, Inc., Boston, MA, 1993); Sambrook et al. (Molecular Cloning, Second Ed., Cold Spring Harbor Laboratory, Plainview, NY, 1989); Maniatis et al. (Molecular Cloning, Cold Spring Harbor Laboratory, Plainview, NY, 1982); Glover (Ed.) (DNA Cloning Vol. I and II, IRL Press, Oxford, UK, 1985); Hames and Higgins (Eds.) (Nucleic Acid Hybridization, IRL Press, Oxford, UK, 1985); and elsewhere.

The DNA sequence in the expression vector is operatively linked to at least one appropriate expression control sequence (*e*.*g*., a constitutive promoter or a regulated promoter) to direct mRNA synthesis. Representative examples of such expression control sequences include promoters of eukaryotic cells or their viruses, as described above. Promoter regions can be selected from any desired gene using CAT (chloramphenicol transferase) vectors, kanamycin vectors, or other vectors with selectable markers. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art, and preparation of certain particularly preferred recombinant expression constructs comprising at least one promoter or regulated promoter operably linked to a nucleic acid encoding a protein or polypeptide according to this disclosure is described herein.

Variants of the polynucleotides of this disclosure are also contemplated. Variant polynucleotides are at least 60%, 65%, 70%, 75%, 80%, 85%, 90%, and preferably 95%, 96%, 97%, 98%, 99%, or 99.9% identical to one of the polynucleotides of defined sequence as described herein, or that hybridizes to one of those polynucleotides of defined sequence under stringent hybridization conditions of 0.015M sodium chloride, 0.0015M sodium citrate at about 65-68°C or 0.015M sodium chloride, 0.0015M sodium citrate, and 50% formamide at about 42°C. The polynucleotide variants retain the capacity to encode a binding domain or fusion protein thereof having the functionality described herein.

The term "stringent" is used to refer to conditions that are commonly understood in the art as stringent. Hybridization stringency is principally determined by temperature, ionic strength, and the concentration of denaturing agents such as formamide. Examples of stringent conditions for hybridization and washing are 0.015M sodium chloride, 0.0015M sodium citrate at about 65-68°C or 0.015M sodium chloride, 0.0015M sodium citrate, and 50% formamide at about 42°C (see Sambrook et ai, Molecular Cloning: A Laboratory Manual, 2nd Ed., Cold Spring Harbor Laboratory, Cold Spring Harbor, N.Y., 1989). More stringent conditions (such as higher temperature, lower ionic strength, higher formamide, or other denaturing agent) may also be used; however, the rate of hybridization will be affected. In instances wherein hybridization of deoxyoligonucleotides is concerned, additional exemplary stringent hybridization conditions include washing in 6x SSC, 0.05% sodium pyrophosphate at 37°C (for 14-base oligonucleotides), 48°C (for 17-base oligonucleotides), 55°C (for 20-base oligonucleotides), and 60°C (for 23-base oligonucleotides).

A further aspect of this disclosure provides a host cell transformed or transfected with, or otherwise containing, any of the polynucleotides or vector/expression constructs of this disclosure. The polynucleotides or cloning/expression constructs of this disclosure are introduced into suitable cells using any method known in the art, including transformation, transfection and transduction. Host cells include the cells of a subject undergoing ex vivo cell therapy including, for example, ex vivo gene therapy. Eukaryotic host cells contemplated as an aspect of this disclosure when harboring a polynucleotide, vector, or protein according to this disclosure include, in addition to a subject's own cells (*e*.*g*., a human patient's own cells), VERO cells, HeLa cells, Chinese hamster ovary (CHO) cell lines (including modified CHO cells capable of modifying the glycosylation pattern of expressed multivalent binding molecules, see US Patent Application Publication No. 2003/01 15614), COS cells (such as COS-7), W138, BHK, HepG2, 3T3, RIN, MDCK, A549, PC12, K562, HEK293 cells, HepG2 cells, N cells, 3T3 cells, Spodoptera frugiperda cells (*e*.*g*., Sf9 cells), Saccharomyces cerevisiae cells, and any other eukaryotic cell known in the art to be useful in expressing, and optionally isolating, a protein or peptide according to this disclosure. Also contemplated are prokaryotic cells, including Escherichia coli, Bacillus subtilis, Salmonella typhimurium, a Streptomycete, or any prokaryotic cell known in the art to be suitable for expressing, and optionally isolating, a protein or peptide according to this disclosure. In isolating protein or peptide from prokaryotic cells, in particular, it is contemplated that techniques known in the art for extracting protein from inclusion bodies may be used. The selection of an appropriate host is within the scope of those skilled in the art from the teachings herein. Host cells that glycosylate the fusion proteins of this disclosure are contemplated.

The term "recombinant host cell" (or simply "host cell") refers to a cell containing a recombinant expression vector. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. Recombinant host cells can be cultured in a conventional nutrient medium modified as appropriate for activating promoters, selecting transformants, or amplifying particular genes. The culture conditions for particular host cells selected for expression, such as temperature, pH and the like, will be readily apparent to the ordinarily skilled artisan. Various mammalian cell culture systems can also be employed to express recombinant protein. Examples of mammalian expression systems include the COS-7 lines of monkey kidney fibroblasts, described by Gluzman (1981 ) Cell 23:175, and other cell lines capable of expressing a compatible vector, for example, the C127, 3T3, CHO, HeLa and BHK cell lines. Mammalian expression vectors will comprise an origin of replication, a suitable promoter and, optionally, enhancer, and also any necessary ribosome binding sites, polyadenylation site, splice donor and acceptor sites, transcriptional termination sequences, and 5'-flanking nontranscribed sequences, for example, as described herein regarding the preparation of multivalent binding protein expression constructs. DNA sequences derived from the SV40 splice, and polyadenylation sites may be used to provide the required nontranscribed genetic elements. Introduction of the construct into the host cell can be effected by a variety of methods with which those skilled in the art will be familiar, including calcium phosphate transfection, DEAE-Dextran-mediated transfection, or electroporation (Davis et al. (1986) Basic Methods in Molecular Biology).

### Cells and Compositions

In one embodiment, the B cell compositions described herein utilize memory B cells at the start of *in vitro* culture and demonstrate longer *in vivo* survival in comparison to naive B cells that undergo the same treatment. In another embodiment, the B cell compositions described herein utilize naive B cells at the start of *in vitro* culture. In one embodiment, the B cell compositions described herein utilize pan B cells at the start of *in vitro* culture. In one embodiment, the starting cell population comprises naive B cells and memory B cells.

In one embodiment, the cell compositions described herein comprise B cells that have been activated/differentiated *in vitro* and transfected to express a protein of interest as described herein. In one embodiment, the compositions comprise B cells that have differentiated into plasma B cells, have been transfected and express one or more proteins of interest. Target cell populations, such as the transfected and activated B cell populations of the present disclosure may be administered either alone, or as a pharmaceutical composition in combination with diluents and/or with other components such as cytokines or cell populations. Briefly, cell compositions of the present disclosure may comprise a differentiated and activated B cell population that has been transfected and is expressing a protein of interest as described herein, in combination with one or more pharmaceutically or physiologically acceptable carriers, diluents or excipients. Such compositions may comprise buffers such as neutral buffered saline, phosphate buffered saline, Lactated Ringer's solution and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present disclosure are preferably formulated for intravenous or subcutaneous administration.

In one embodiment, a cell composition is assessed for purity prior to administration. In another embodiment, a cell composition is tested for robustness of therapeutic agent production. In one embodiment, a cell composition is tested for sterility. In another embodiment, a cell composition is screened to confirm it matches the recipient subject.

In one embodiment, a cell composition is stored and/or shipped at 4°C. In another embodiment, a cell composition is frozen for storage and/or shipment. A cell composition may be frozen at, *e*.*g*., -20°C or -80°C. In one embodiment, a step of freezing a cell composition comprises liquid nitrogen. In one embodiment, a cell composition is frozen using a controlled rate freezer. Accordingly, methods described herein may further include a thawing step.

### Methods of Use

Due to the longer *in vivo* survival demonstrated by the cell compositions starting from memory B cell populations, the cell compositions described herein are particularly well suited to the long term *in vivo* delivery of a therapeutic agent. However, the B cell compositions from naive or pan B cell starting populations are also useful for *in vivo* delivery of a therapeutic agent. In particular embodiments, the cell compositions are used in methods of treating and/or preventing chronic diseases and disorders.

Cell compositions described herein may be administered in a manner appropriate to the disease or disorder to be treated or prevented. The quantity and frequency of administration will be determined by such factors as the condition of the patient, and the type and severity of the patient's disease, although appropriate dosages may be determined by clinical trials.

When "an effective amount", "an anti-tumor effective amount", "a tumor-inhibiting effective amount", or "therapeutic amount" is indicated, the precise amount of the compositions of the present disclosure to be administered can be determined by a physician with consideration of individual differences in age, weight, tumor size, extent of infection or metastasis, and condition of the patient (subject). B cell compositions may also be administered multiple times at an appropriate dosage(s). The cells can be administered by using infusion techniques that are commonly known in immunotherapy (see, e.g., Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly. The treatment may also be adjusted after measuring the levels of a therapeutic agent (*e*.*g*., a gene or protein of interest) in a biological sample (*e*.*g*., body fluid or tissue sample) can also be used to assess the treatment efficacy, and the treatment may be adjusted accordingly to increase or decrease. Typically, in related adoptive immunotherapy studies, antigen-specific T cells are administered approximately at 2 × 10⁹ to 2 × 10¹¹ cells to the patient. (See, *e*.*g*., U.S. Pat. No. 5,057,423). In some aspects of the present disclosure, lower numbers of the transfected B cells of the present disclosure, in the range of 10⁶/kilogram (10⁶-10¹¹ per patient) may be administered. In certain embodiments, the B cells are administered at 1 × 10⁴, 5 × 10⁴, 1 × 10⁵, 5 × 10⁵, 1 × 10⁶, 5 × 10⁶, 1 × 10⁷, 5 × 10⁷, 1 × 10⁸ , 5 × 10⁸, 5 × 10⁹, 1 × 10¹⁰, 5 × 10¹⁰, 1 × 10¹¹, 5 × 10¹¹, or 1 × 10¹² cells to the subject. B cell compositions may be administered multiple times at dosages within these ranges. The cells may be autologous or heterologous to the patient undergoing therapy. If desired, the treatment may also include administration of mitogens (*e*.*g*., PHA) or lymphokines, cytokines, and/or chemokines (*e*.*g*., GM-CSF, IL-4, IL-13, Flt3-L, RANTES, MIP1α, etc.) as described herein to enhance induction of an immune response and engraftment of the infused B cells.

The administration of the subject compositions may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous (i.v.) injection, or intraperitoneally. In one embodiment, the B cell compositions of the present disclosure are administered to a patient by intradermal or subcutaneous injection. In another embodiment, the B cell compositions as described herein are preferably administered by i.v. injection. The compositions of B cells may be injected directly into a tumor, lymph node, bone marrow or site of infection.

In yet another embodiment, the pharmaceutical composition can be delivered in a controlled release system. In one embodiment, a pump may be used (see Langer, 1990, Science 249:1527-1533; Sefton 1987, CRC Crit. Ref. Biomed. Eng. 14:201 ; Buchwald et al., 1980; Surgery 88:507; Saudek et al., 1989, N. Engl. J. Med. 321 :574). In another embodiment, polymeric materials can be used (see Medical Applications of Controlled Release, 1974, Langer and Wise (eds.), CRC Pres., Boca Raton, Fla.; Controlled Drug Bioavailability, Drug Product Design and Performance, 1984, Smolen and Ball (eds.), Wiley, New York; Ranger and Peppas, 1983; J. Macromol. Sci. Rev. Macromol. Chem. 23:61 ; see also Levy et al., 1985, Science 228:190; During et al., 1989, Ann. Neurol. 25:351 ; Howard et al., 1989, J. Neurosurg. 71 :105). In yet another embodiment, a controlled release system can be placed in proximity of the therapeutic target, thus requiring only a fraction of the systemic dose (see, *e*.*g*., Medical Applications of Controlled Release, 1984, Langer and Wise (eds.), CRC Pres., Boca Raton, Fla., vol. 2, pp. 1 15-138).

The B cell compositions of the present disclosure may also be administered using any number of matrices. Matrices have been utilized for a number of years within the context of tissue engineering (see, *e*.*g*., Principles of Tissue Engineering (Lanza, Langer, and Chick (eds.)), 1997. The present disclosure utilizes such matrices within the novel context of acting as an artificial lymphoid organ to support and maintain the B cells. Accordingly, the present disclosure can utilize those matrix compositions and formulations which have demonstrated utility in tissue engineering. Accordingly, the type of matrix that may be used in the compositions, devices and methods of the disclosure is virtually limitless and may include both biological and synthetic matrices. In one particular example, the compositions and devices set forth by U.S. Patent Nos: 5,980,889; 5,913,998; 5,902,745; 5,843,069; 5,787,900; or 5,626,561 are utilized. Matrices comprise features commonly associated with being biocompatible when administered to a mammalian host. Matrices may be formed from natural and/or synthetic materials. The matrices may be nonbiodegradable in instances where it is desirable to leave permanent structures or removable structures in the body of an animal, such as an implant; or biodegradable. The matrices may take the form of sponges, implants, tubes, telfa pads, fibers, hollow fibers, lyophilized components, gels, powders, porous compositions, or nanoparticles. In addition, matrices can be designed to allow for sustained release seeded cells or produced cytokine or other active agent. In certain embodiments, the matrix of the present disclosure is flexible and elastic, and may be described as a semisolid scaffold that is permeable to substances such as inorganic salts, aqueous fluids and dissolved gaseous agents including oxygen.

A matrix is used herein as an example of a biocompatible substance. However, the current disclosure is not limited to matrices and thus, wherever the term matrix or matrices appears these terms should be read to include devices and other substances which allow for cellular retention or cellular traversal, are biocompatible, and are capable of allowing traversal of macromolecules either directly through the substance such that the substance itself is a semi-permeable membrane or used in conjunction with a particular semi-permeable substance.

In certain embodiments of the present disclosure, B cells transfected and activated using the methods described herein, or other methods known in the art, are administered to a patient in conjunction with (e.g. before, simultaneously or following) any number of relevant treatment modalities, including but not limited to treatment with agents such as antiviral agents, chemotherapy, radiation, immunosuppressive agents, such as cyclosporin, bisulfin, bortezomib, azathioprine, methotrexate, mycophenolate, and FK506, antibodies, or other immunoablative agents such as CAMPATH, anti-CD3 antibodies or other antibody therapies, cytoxin, fludaribine, cyclosporin, FK506, rapamycin, mycophenolic acid, steroids, FR901228, cytokines, and irradiation. These drugs inhibit either the calcium dependent phosphatase calcineurin (cyclosporine and FK506), the proteasome (bortezomib), or inhibit the p70S6 kinase that is important for growth factor induced signaling (rapamycin). (Liu et al., Cell 66:807-815, 1991 ; Henderson et al., Immun. 73:316-321 , 1991 ; Bierer et al., Curr. Opin. Immun. 5:763-773, 1993; Isoniemi (supra)). In a further embodiment, the cell compositions of the present disclosure are administered to a patient in conjunction with (e.g. before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In one embodiment, the cell compositions of the present disclosure are administered following B-cell ablative therapy such as agents that react with CD20, *e*.*g*. Rituxan^{®}. In one embodiment, the cell compositions of the present disclosure are administered following B cell ablative therapy using an agent such as bortezomib. For example, in one embodiment, subjects may undergo standard treatment with high dose chemotherapy followed by peripheral blood stem cell transplantation. In certain embodiments, following the transplant, subjects receive an infusion of the expanded immune cells of the present disclosure. In an additional embodiment, expanded cells are administered before or following surgery.

The dosage of the above treatments to be administered to a patient will vary with the precise nature of the condition being treated and the recipient of the treatment. The scaling of dosages for human administration can be performed according to art-accepted practices.

The transfected B cell compositions of the disclosure, particularly B cells transfected to express a particular antibody of interest, can be used in the treatment or prevention of various infectious diseases, cancers, degenerative diseases and immunological disorders.

Compositions comprising the transfected B cells as described herein may be used in treatment of any of a variety of infectious diseases caused by infectious organisms, such as viruses, bacteria, parasites and fungi. Infectious organisms may comprise viruses, (*e.g*., RNA viruses, DNA viruses, human immunodeficiency virus (HIV), hepatitis A, B, and C virus, herpes simplex virus (HSV), cytomegalovirus (CMV) Epstein-Barr virus (EBV), human papilloma virus (HPV)), parasites (*e.g*., protozoan and metazoan pathogens such as Plasmodia species, Leishmania species, Schistosoma species, Trypanosoma species), bacteria (*e.g*., Mycobacteria, in particular, M. tuberculosis, Salmonella, Streptococci, E. coli, Staphylococci), fungi (*e.g*., Candida species, Aspergillus species), Pneumocystis carinii, and prions (known prions infect animals to cause scrapie, a transmissible, degenerative disease of the nervous system of sheep and goats, as well as bovine spongiform encephalopathy (BSE), or "mad cow disease", and feline spongiform encephalopathy of cats. Four prion diseases known to affect humans are (1) kuru, (2) Creutzfeldt-Jakob Disease (CJD), (3) Gerstmann-Straussler-Scheinker Disease (GSS), and (4) fatal familial insomnia (FFI)). As used herein "prion" includes all forms of prions causing all or any of these diseases or others in any animals used-and in particular in humans and domesticated farm animals. Illustrative infectious diseases include, but are not limited to, toxoplasmosis, histoplasmosis, CMV, EBV, coccidiomycosis, tuberculosis, HIV, and the like.

In certain embodiments, the transfected B cell compositions as described herein may also be used for the prevention or treatment of a variety of cancers. In this regard, in certain embodiments, the compositions comprising transfected B cells are useful for preventing or treating melanoma, non-Hodgkin's lymphoma, Hodgkin's disease, leukemia, plasmocytoma, sarcoma, glioma, thymoma, breast cancer, prostate cancer, colo-rectal cancer, kidney cancer, renal cell carcinoma, uterine cancer, pancreatic cancer, esophageal cancer, brain cancer, lung cancer, ovarian cancer, cervical cancer, testicular cancer, gastric cancer, esophageal cancer, multiple myeloma, hepatoma, acute lymphoblastic leukemia (ALL), acute myelogenous leukemia (AML), chronic myelogenous leukemia (CML), and chronic lymphocytic leukemia (CLL), or other cancers.

In one embodiment, the transfected B cells may also be used in the treatment of immunological disorders such as acquired immune deficiency syndrome (AIDS), agammaglobulinemia, hypogammaglobulinemia, other immunodeficiencies, immunosuppression, and severe combined immunodeficiency disease (SCID).

In one embodiment, the transfected B cells as described herein may also be used in the treatment of autoimmune diseases such as, but not limited to, rheumatoid arthritis, multiple sclerosis, insulin dependent diabetes, Addison's disease, celiac disease, chronic fatigue syndrome, inflammatory bowel disease, ulcerative colitis, Crohn's disease, Fibromyalgia, systemic lupus erythematosus, psoriasis, Sjogren's syndrome, hyperthyroid ism/Graves disease, hypothyroidism/Hashimoto's disease, Insulin-dependent diabetes (type 1), Myasthenia Gravis, endometriosis, scleroderma, pernicious anemia, Goodpasture syndrome, Wegener's disease, glomerulonephritis, aplastic anemia, paroxysmal nocturnal hemoglobinuria, myelodysplastic syndrome, idiopathic thrombocytopenic purpura, autoimmune hemolytic anemia, Evan's syndrome, Factor VIII inhibitor syndrome, systemic vasculitis, dermatomyositis, polymyositis and rheumatic fever. Thus, in one embodiment, the methods herein include methods for treating a disease comprising administering to a subject or patient in need thereof a therapeutically effective amount of the compositions comprising the transfected B cells as described herein, thereby treating the disease.

In one embodiment, the transfected B cells as described herein may also be used in the treatment of enzyme deficiency diseases and disorders such as, but not limited to, MPS I, MPS II, MPS III, MP IV, MPS V, MPS VI, MPS VII, lysosomal storage disorders, Nieman-pick disease (types A, B and C), Guacher's disease (types I, II and III), Tay-Sachs disease and Pompe disorder.

### EXAMPLES

### EXAMPLE 1

### IN VIVO SURVIVAL OF B CELLS ACTIVATED AND DIFFERENTIATED IN VITRO

In order to determine whether the phenotype of a population of B cells prior to *in vitro* activation and differentiation affects the amount of time the B cells survive *in vivo,* a mouse model was used to compare the *in vivo* survival of two populations of *in vitro* activated human B cells.

### B Cell Isolation

The first population of B cells comprised CD19+ pan-B cells, and the second population of B cells comprised memory B cells. Fixed pan-B cells were used as a negative control. PBMCs were isolated from human whole blood samples using FICOLL gradient separation. Pan-B cells were isolated from the PBMCs by negative selection using a Pan-B cell isolation kit according to manufacturer's instructions (Miltenyi Biotec). Briefly, the cell suspension was centrifuged at 300g for 10 minutes after counting. The supernatant was discarded, and the cells were resuspended at 10⁸ cells/400 µl cold buffer for a total concentration of 4.5 × 10⁸ cells/1.8 ml. Next 450 µl of the B cell biotinylated antibody cocktail was added to the cells and incubated for 40 minutes at 4°C. 1.25 ml of cold buffer and 900 µl of Anti-Biotin MicroBeads were added and incubated for 15 minutes at 4°C. Cells were washed by adding 10 ml buffer and centrifuging at 300g for 10 minutes. The supernatant was aspirated completely, and cells were resuspended in 4.5 ml cold buffer. Using magnetic separation, non-B cells were depleted by applying the cell suspension to a MACS^{®} Column in a magnetic MACS Separator. Following two washes of the column, the effluent contained the B cell fraction (CD19+ pan-B cells).

Isolation of memory B cells utilized an additional step of positive selection for CD27 with a memory B cell isolation kit according to manufacturer's instructions (Miltenyi Biotec). The B cell fraction obtained above was centrifuged at 300g for 10 minutes, and the supernatant was aspirated completely. The cells were resuspended in 450 µl of buffer, and 450 µl of CD27 MicroBeads were added and incubated for 15 minutes at 4°C. Cells were washed by adding 20 ml buffer and centrifuging at 300g for 10 minutes. The supernatant was aspirated completely, and cells were resuspended in 225 ml of cold buffer. Using magnetic separation, CD27+ memory B cells were retained by applying the cell suspension to a MACS^{®} Column in a magnetic MACS Separator. Following three washes of the column, the column was removed from the magnetic field and labeled cells were flushed out of the column using 2.5 ml of buffer and pushing a plunger into the column to obtain the memory B cell fraction (CD27+ B cells).

Alternatively, memory B cells were isolated by depletion of CD3+ and CD56+ cells, followed by positive selection for CD27+ cells as described above.

### In Vitro Culture

Both the pan-B cell population and the memory B cell population were cultured *in vitro* prior to administration to mice. The CD19+ pan-B cells were differentiated using a 3-stage culture system. The base culture media comprised Iscove's Modified Dulbecco's Medium (IMDM), 10% fetal bovine serum (FBS) and 50 µg/ml of transferrin. During the first stage of the culture system, the cells were exposed for 5 days to 10 µg/ml CpG, 50 ng/ml IL-10 and 10 ng/ml IL-15. For the next stage, media was replaced, and cells were cultured in media containing 20 U/ml IL-2, 50 ng/ml IL-10, 10 ng/ml IL-15, 50 ng/ml IL-6 and 1 µg/ml anti-CD40L antibody. After 3 days of culture in the stage 2 media, the media was replaced, and the cells cultured for an additional 3 days to induce differentiation into plasmablasts and plasma cells. Specifically, stage 3 media contained 10 ng/ml IL-15, 50 ng/ml IL-6, 500 U/ml IFN-alpha, 20 ng/ml HFG and 100 µg/ml hyaluronic acid.

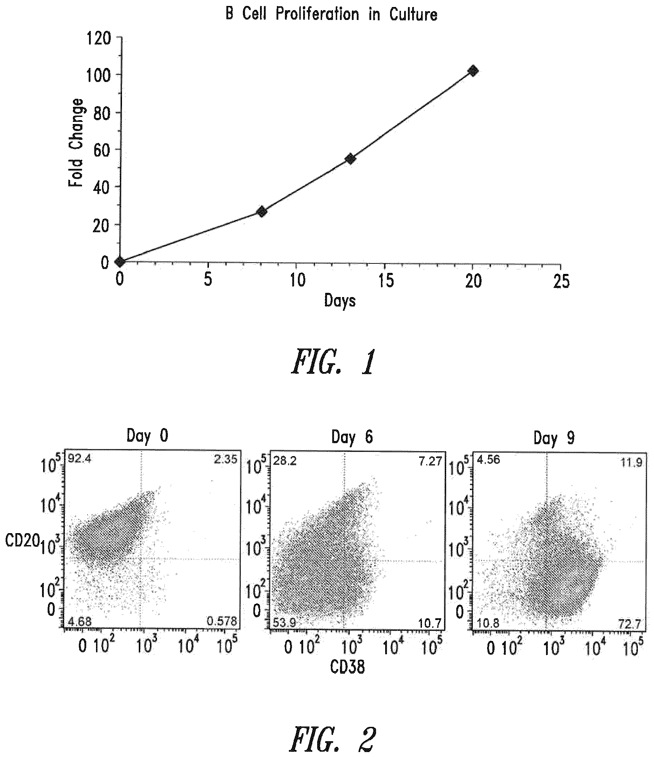

Following positive selection of CD27+ cells, the memory B cells were cultured in IMDM with 10% FBS, 1% penicillin-streptomycin solution, transferrin and insulin. Histidine(his)-tagged CD40L and anti-poly-his mAb were also added to the culture medium. Alternatively, a multimeric CD40L or a helper cell-expressed CD40L could be utilized. The cytokines added to the culture medium on Day 0 were IL-2, IL-10, and IL-15. P-class CpG oligodeoxynucleotides (P-ODNs) were also added to the culture medium. Exemplary expansion of B cells cultured with IL-2, IL-10, IL-15 and CpG is shown in Figure 1. On *in vitro* culture Day 3, cells were harvested, washed with base medium (non-supplemented IMDM), centrifuged, and resuspended in IMDM supplemented with IL-2, IL-6, IL-10 and IL-15. On *in vitro* culture Day 6, cells were harvested, washed, centrifuged, and resuspended in IMDM supplemented with IFN-αA/D, IL-6 and IL-10. On Day 9 of *in vitro* culture, cells were harvested, washed twice, and resuspended in either flow cytometry staining buffer solution or freezing medium for storage. Cells to be frozen for storage were placed at -80°C overnight before transferring into liquid nitrogen for long-term storage.

The phenotype of the *in vitro* cultured cells was verified by flow cytometry. Representative data of the CD20 and CD38 phenotypes of B cells cultured *in vitro* using conditions to promote differentiation of B cells is shown in Figure 2. Both populations of B cells, pan-B cells and memory B cells, demonstrated similar phenotype kinetics. As shown in Figure 2, the phenotype of the majority of cells at Day 0 is CD20+, CD38-. After 7 days in culture to promote B cell differentiation, the cells are mostly CD20-, CD38+.

### In Vivo Survival in Mice

Following *in vitro* culture, frozen B cells were thawed and stained with the near infrared fluorescent dye DiOC₁₈(7) (DiR). In order to examine the *in vivo* survival of the *in vitro* cultured B cells, NOD *scid* gamma (NSG) mice (The Jackson Laboratory) were utilized so that the human B cells were not killed and cleared by a host immune system. Additionally, relevant mouse and human homing chemokines and receptors, including CXCR4 and CXCL12/SDF-1, are cross-reactive, so that the human cells can migrate in the mouse. Three groups of four NSG mice received 5 × 10⁶ B cells via iv injection. One group of 4 NSG mice received pan-B cells, another group of 4 NSG mice received memory B cells, and the control group of 4 NSG mice received fixed CD19+ B-cells. The pan-B cells and memory B cells were centrifuged and resuspended in PBS for injection. The control cells were placed in 3% paraformaldehyde in PBS for 15 minutes, washed two times in PBS, and resuspended in PBS for injection.

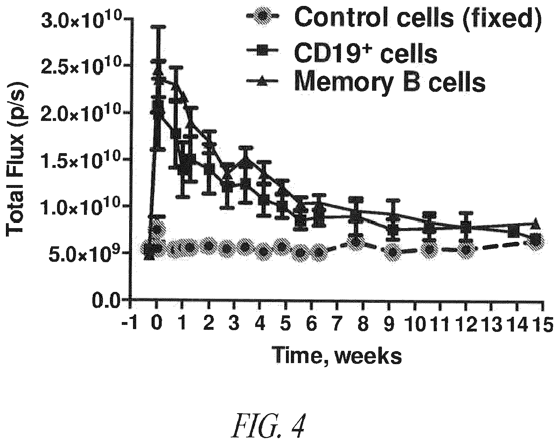

Mice were examined over the course of the study using IVISO. One mouse from each treatment group was imaged at each time point according to the imaging schedule in Table 1 below, and the pattern was repeated over the course of the study. Mice were imaged 2 days prior to the injection to establish a baseline level of fluorescence. The IVISO signal was then measured every 8 hours for the first 10 days, and then every 5 days thereafter until signal was lost or the study ended. The study was originally expected to end at Day 45 post injection; however a large number of cells were persisting at Day 41 (Figures 3 and 4). Two mice from each group were sacrificed on Day 45 as originally planned, and the remaining mice (two from each group) continued to be imaged every 5 days until Day 100. As can be seen in Figure 3, the memory B cell population was still widely present in the mice at Day 81, while the pan-B cell population was similar to the control sample.

**Table 1. Imaging Schedule**

| Time point! Mouse | -2 days | 1 hour | 8 hours | 16 hours | 24 hours | 49 hours | 56 hours | 64 hours | 72 hours |
|---|---|---|---|---|---|---|---|---|---|
| 1 | X | X | | | | X | | | |
| 2 | | | X | | | | X | | |
| 3 | | | | X | | | | X | |
| 4 | | | | | X | | | | X |

These results demonstrate that the memory B cell culture conditions induced cell proliferation and phenotypic transition and resulted in improved *in vivo* survival. In particular, it is important to note that while the NSG mouse model provides relevant migration signals for the cells, the survival signals (*e.g*., B cell activating factor (BAFF) and IL-6) are not cross-reactive, so the B cells do not survive as long as they would in a human.

Accordingly, the present methods can be used to produce B cells for long term delivery of a therapeutic agent, such as a specific antibody or other therapeutic protein. No methods described in the art have differentiated transduced memory B cells into plasmablasts and plasma cells *in vitro* prior to *in vivo* administration for long term delivery of a therapeutic agent.

### EXAMPLE 2

### TRANSDUCTION OF IN VITRO ACTIVATED AND DIFFERENTIATED B CELLS

In order to optimize culture conditions and demonstrate that *in vitro* cultured memory B cells can be transduced, VSV-G pseudotyped lentiviral vectors were utilized to introduce a gene of interest into B cells.

Memory B cells were isolated as described in the previous example. Following positive selection of CD27+ cells, the memory B cells were cultured in IMDM with 10% FBS, 1% penicillin-streptomycin solution, transferrin and insulin. His-tagged CD40L and anti-poly-his mAb were also added to the culture medium. The cytokines added to the culture medium on Day 0 were IL-2, IL-10, and IL-15. P-class CpG oligodeoxynucleotides (P-ODNs) were also added to the culture medium. On *in vitro* culture Day 3, cells were harvested, washed with base medium (non-supplemented IMDM), centrifuged, and resuspended in IMDM supplemented with IL-2, IL-6, IL-10 and IL-15. On *in vitro* culture Day 5, cells were harvested, washed, centrifuged and resuspended in IMDM supplemented with protamine sulfate for viral transduction with lentivirus. Lentivirus harboring GFP was added to cells at a multiplicity of infection (MOI) of 3 and incubated overnight. On *in vitro* culture Day 6, cells were harvested, washed, centrifuged, and resuspended in IMDM supplemented with IFN-αA/D, IL-6 and IL-10. On Day 9 of *in vitro* culture, cells were harvested, washed twice, and resuspended in either flow cytometry staining buffer solution or freezing medium for storage. Cells to be frozen for storage were placed at -80°C overnight before transferring into liquid nitrogen for long-term storage.

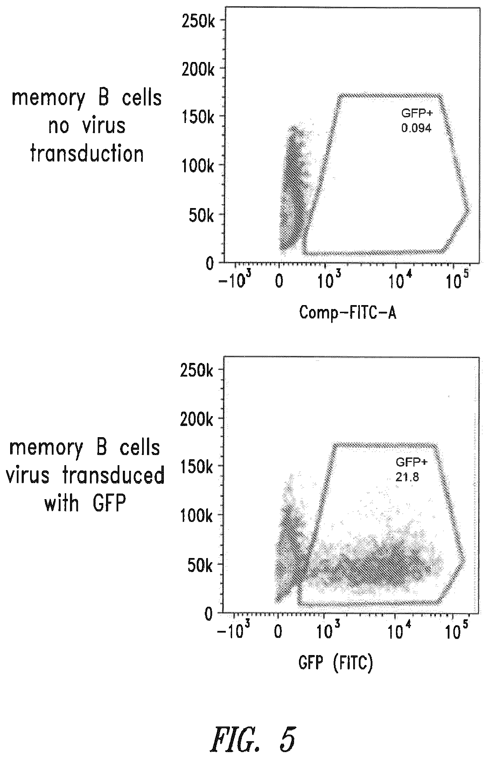

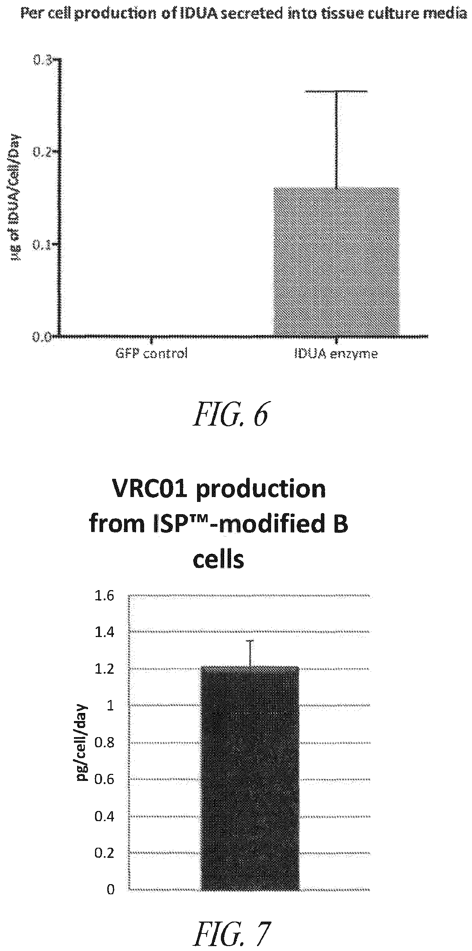

As shown in Figure 5, flow cytometry was used to observe the presence of GFP (x-axis). Forward scatter is shown on the y-axis. Using the cell culture system described above for transduction with GFP lentivirus, B cells expressing IDUA (Figure 6) and VRC01 (Figure 7) were also generated. Recombinant protein was detected in the cell culture media of transduced B cells.

For the first time, by using the methods described herein for the transduction of memory B cells followed by *in vitro* culture, differentiated B cells secreting a therapeutic protein were generated.

### EXAMPLE 3

### TRANSFECTION OF IN VITRO ACTIVATED AND DIFFERENTIATED B CELLS

In order to optimize culture conditions and demonstrate that *in vitro* cultured memory B cells can be transfected using electroporation, transposon vectors were utilized to introduce a gene of interest into B cells.

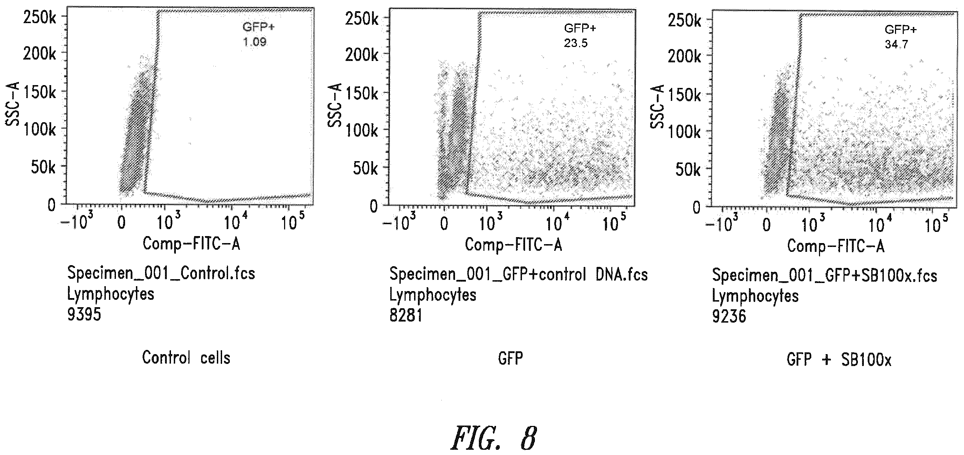

Memory B cells were isolated from PBMCs as described in the previous examples. Cells were electroporated on day 2 of *in vitro* culture using the 4D-Nucleofactor^{™} System (Lonza). Memory B cells were cultured in IMDM with 10% FBS, 1% penicillin-streptomycin solution, transferrin and insulin. His-tagged CD40L and anti-poly-his mAb were also added to the culture medium. The cytokines added to the culture medium on Day 0 were IL-2, IL-10, and IL-15. P-class CpG oligodeoxynucleotides (P-ODNs) were also added to the culture medium. As mentioned, B cells were electroporated on Day 2. Additionally, stimulation of B cells with IL-2, IL-4, IL-10 and CD40L supports effective electroporation on following 2 days of stimulation. On *in vitro* culture Day 3, cells were harvested, washed with base medium (non-supplemented IMDM), centrifuged, and resuspended in IMDM supplemented with IL-2, IL-6, IL-10 and IL-15. On *in vitro* culture Day 6, cells were harvested, washed, centrifuged, and resuspended in IMDM supplemented with IFN-α A/D, IL-6 and IL-10. On Day 9 of *in vitro* culture, cells were harvested, washed twice, and resuspended in either flow cytometry staining buffer solution or freezing medium for storage. Cells were electroporated with an empty transposon vector (control, Figure 8A), a transposon harboring GFP in the absence of transposase (Figure 8B), and a transposon harboring GFP along with the transposase SB100x (Figure 8C).

Thus, by using the methods described herein for the transfection of memory B cells followed by *in vitro* culture, differentiated B cells secreting a therapeutic protein were generated.

### EXAMPLE 4

### IN VIVO SURVIVAL OF MODIFIED CELLS IN A HUMANIZED MOUSE MODEL

In order to determine whether the modified memory B cells described in the Example 1 survive longer in a more humanized mouse model, CD34+ humanized mice (The Jackson Laboratory) may be used. To generate the CD34+ humanized mice, NSG mice are grafted with CD34+ hematopoietic stem cells (HSCs) from the same donor as the B cells.

The control, pan-B cell and memory B cell populations are prepared as described in Example 1. Three groups (control, pan-B cell and memory B cell) of four CD34+ humanized mice receive 5 × 10⁶ stained B cells via iv injection. Mice are examined over the course of the study using IVIS^{®} as described above. One mouse from each group is imaged every 8 hours for the first 10 days, and then every 5 days thereafter until the end of the study.

### EXAMPLE 5

### ENHANCED IN VITRO EXPANSION AND DIFFERENTIATION OF MEMORY B CELLS

In order to determine whether switched memory B cells have greater proliferative potential than pan (CD27+) memory B cells, a switched memory B cell population was obtained prior to expansion and cell differentiation. Further, culture conditions aimed to facilitate increased expansion of the memory cells were tested.

Isolation of pan (CD27+) memory B cells or switched memory B cells was performed according to the manufacturer's instructions (Miltenyi Biotec). Following purification of the memory cell populations, the cells were cultured in IMDM with 10% FBS, 1% penicillin-streptomycin solution, transferrin and insulin. In order to facilitate expansion, the cells were combined with 0.5ug/ml of His-tagged CD40L and anti-poly-his mAb, (5ng/ml) IL-2, (2ng/ml) IL-4, (40ng/ml) IL-10 were also added to the culture medium. The cells were supplemented with new medium every 3-4 days as needed. Depending on the rate of cell proliferation, the cells are expanded for 6-14 days. The cells are then transferred to conditions to induce differentiation.

Specifically, the cell media was then changed to include His-tagged CD40L and anti-poly-his mAb, the cytokines IL-2, IL-10, and IL-15 and the P-class CpG oligodeoxynucleotides (P-ODNs). After 3-4 days of culture in this media formulation the cells were harvested, washed with base medium (non-supplemented IMDM), centrifuged, and resuspended in IMDM supplemented with IL-2, IL-6, IL-10 and IL-15. After 3 days of culture in this media formulation, the cells were harvested, washed, centrifuged and resuspended in IMDM supplemented with IFN-αA/D, IL-6 and IL-10. On Day 9 or 10 of *in vitro* culture, cells were harvested, washed twice, and resuspended in either flow cytometry staining buffer solution or freezing medium for storage. Cells to be frozen for storage were placed at -80°C overnight before transferring into liquid nitrogen for long-term storage.

As demonstrated, the inclusion of conditions to support proliferation prior to differentiation inducing conditions increased the final yield of differentiated cells.

### EXAMPLE 6

### ALTERNATIVE MEANS TO DIFFERENTIATE MEMORY B CELLS FOLLOWING EXPANSION

As an alternative method to differentiate the cells, a culture system utilizing IL-21 was tested. The culture conditions were similar to those described by Cocco et al. (J Immunology 189:5773-5785, 2012). Specifically, memory B cells were cultured at 2.5 × 10⁵/ml with IL-2 (20 U/ml), IL-21 (50 ng/ml), F(ab')₂ goat anti-human IgM and IgG (10 µg/ml) in the presence of CD40 ligand for 3 days. Cells were then re-seeded at 10⁵/ml in media supplemented with IL-2 (20 U/ml), IL-21 (50 ng/ml), HybridoMax hybridoma growth supplement (11 µl/ml), Lipid Mixture 1, chemically defined and MEM amino acids solution (1X final concentration) for an additional 3 days. Cells were reseeded at 2.5 × 10⁵/ml in media supplemented with IL-6 (10 ng/ml), IL-21 (50 ng/ml), IFN-a (100 U/ml), HybridoMax hybridoma growth supplement (11 µl/ml), Lipid Mixture 1 and chemically defined and MEM amino acid solution.

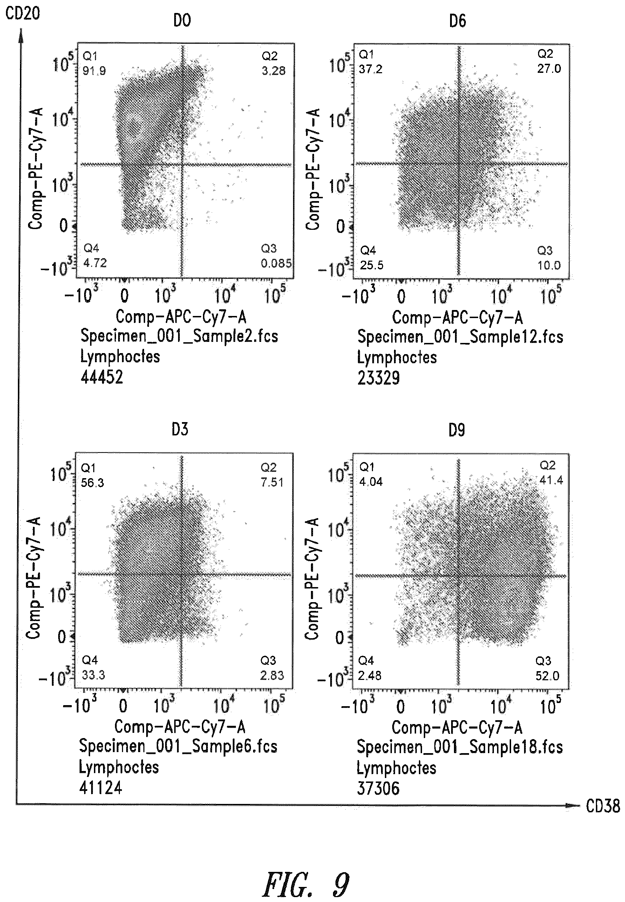

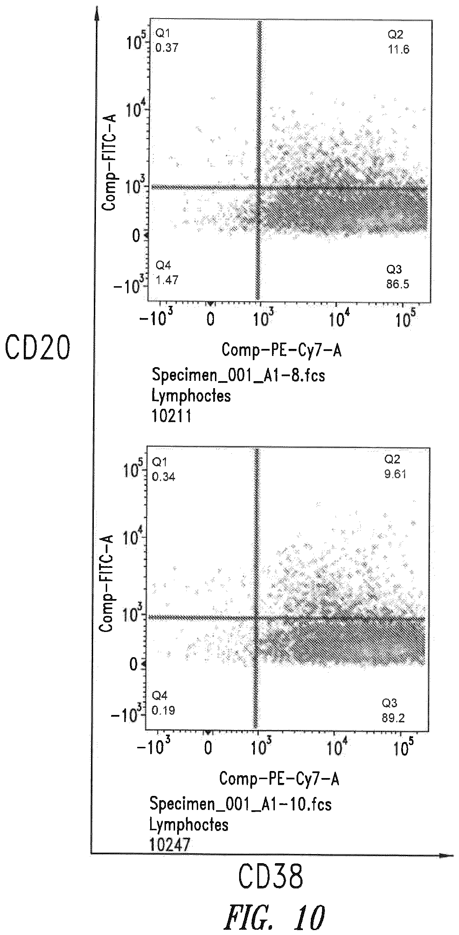

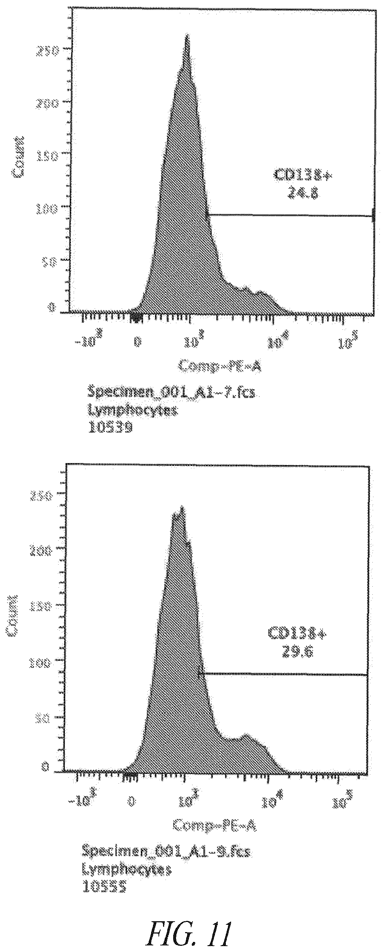

The differentiation of the cells was monitored using flow cytometry observing CD20 and CD38 expression over the course of 10 days (Figure 9). Using the IL-21 culture system, differentiation of switched memory B cells and pan (CD27+) memory B cells resulted in similar expression of CD20 and CD38 in the final population as shown in Figure 10. Additionally, the expression of CD138 was similar when starting with switched memory B cells and pan memory B cells (Figure 11).

### EXAMPLE 7

### IN VITRO EXPANSION OF MEMORY B CELLS

In order to optimize culture conditions for expansion of memory cells, the culture conditions described above in Example 5 utilizing a combination of CD40L, IL-2, IL-4, and IL-10 were compared to culture conditions that also included IL-15 and IL-21.

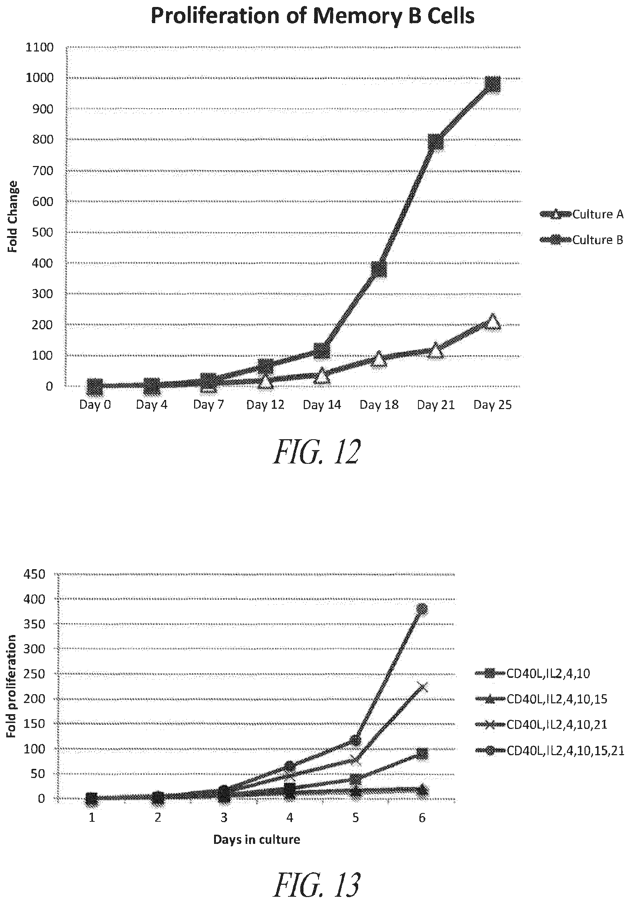

Pan (CD27+) memory B cells were isolated as described above. Following purification of the memory cells, the cells were cultured in IMDM with 10% FBS, 1% penicillin-streptomycin solution, transferrin and insulin. For expansion, the cells were cultured with either a combination of 0.5 µg/ml of His-tagged CD40L and anti-poly-his mAb, 5 ng/ml IL-2, 2 ng/ml IL-4, and 40 ng/ml IL-10 (Culture A) or a combination of 0.5 µg/ml of His-tagged CD40L and anti-poly-his mAb, 5 ng/ml IL-2, 2 ng/ml IL-4, 40ng/ml IL-10, 100 ng/ml IL-15, and 100 ng/ml IL-21 (Culture B). The cells were supplemented with new medium every 3-4 days as needed. Cell counts were performed on days 0, 4, 7, 12, 14, 18, 21 and 25 in order to determine the fold change in cell number. As depicted in Figure 12 and demonstrated below in Table 2, the addition of IL-15 and IL-21 to the cell culture greatly increased the proliferation rate of the memory cells.

**Table 2. Fold change in number of memory B cells**

| | Day 0 | Day 4 | Day 7 | Day 12 | Day 14 | Day 18 | Day 21 | Day 25 |
|---|---|---|---|---|---|---|---|---|
| Culture A | 1 | 2.67 | 7.94 | 20.87 | 39.31 | 91.83 | 120.11 | 213.79 |
| Culture B | 1 | 3.86 | 17.58 | 65.69 | 117.72 | 381.88 | 794.48 | 981.98 |

### EXAMPLE 8

### IN VITRO EXPANSION OF PAN B CELLS

In order to examine the effects of IL-15 and IL-21 on the expansion of pan B cells, *in vitro* culture conditions that included IL-15 and IL-21 alone and in combination were tested.

B cells were isolated from PBMC and grown in media containing CD40L, a CD40L crosslinking agent, IL-2, IL-4, IL-10 and IL-15 and/or IL-21. Accordingly, one B cell population was cultured with CD40L, a CD40L crosslinking agent, IL-2, IL-4, IL-10 and IL-15; another population was cultured with CD40L, a CD40L crosslinking agent, IL-2, IL-4, IL-10 and IL-21; and a third population was cultured with CD40L, a CD40L crosslinking agent, IL-2, IL-4, IL-10, IL-15 and IL-21. Cell counts were taken using an automated cell counter. As shown in Figure 13, the addition of both IL-15 and IL-21 in combination significantly enhanced proliferation of the B cells. In contrast, addition of only IL-15 reduced proliferation, while addition of IL-21 led to a modest increase in proliferation.

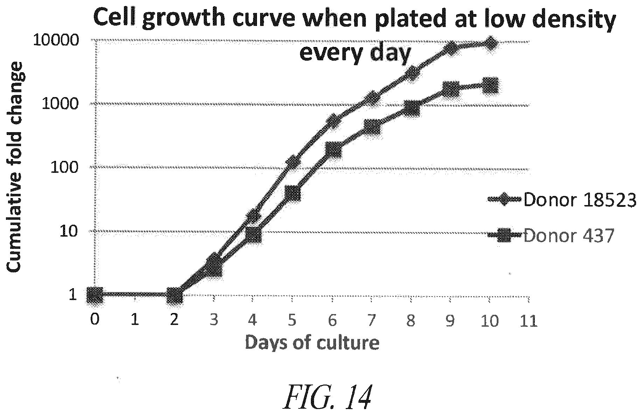

Next, the expansion potential of pan B cells cultured for a time period of 10 days with a combination of IL-15 and IL-21 was examined. B cells were grown in the presence of CD40L, a CD40L crosslinking agent, IL-2, IL-4, IL-10, IL-15, and IL-21 in 24-well cell culture plates. Media was changed daily. The data shown in Figure 14 demonstrates the capacity of the cytokine cocktail described herein to promote high level proliferation of the B cells at a low cell density and in the absence of feeder cells.

### EXAMPLE 9

### MIGRATION OF IN VITRO EXPANDED PAN B CELLS

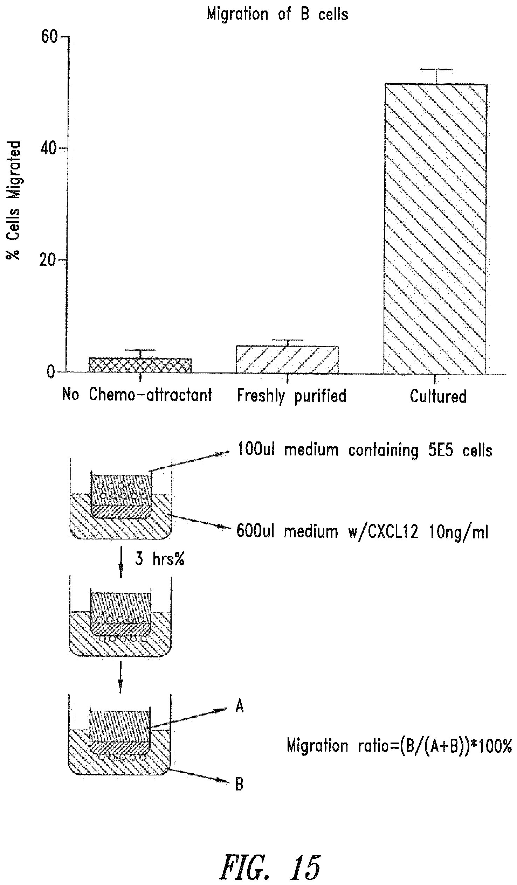

A further experiment was performed in order to study the migratory capacity of the expanded B cells. The assay was conducted using two-chambered culture vessels, in which the chambers are connected (*e.g*., a Transwell^{®} plate). B cells were seeded in one chamber and the other chamber was loaded with 100 ng/mL of CXCL12, which is a chemoattractant that draws B cells to the bone marrow. After allowing the B cells to migrate for 3 hours, B cells were collected from the second well and counted. A negative control was used in which no CXCL12 was added to the second chamber. For a comparison, freshly purified/uncultured B cells were also used. The test group was B cells that were exposed to the culture system described in Example 8 for 6 days. On average, 51.2% of the cultured B cells migrated toward the CXCL12 chamber. The data shown in Figure 15 demonstrates that exposure to the culture system comprising CD40L, a CD40L crosslinking agent, IL-2, IL-4, IL-10, IL-15, and IL-21 greatly enhanced the migratory capacity of the B cells. This finding demonstrates that a starting population of pan B cells is migratory following the *in vitro* culture system described herein, so that the B cells are able to migrate to survival niches, complete differentiation, and receive signals supporting long-term survival following *in vivo* administration.

The various embodiments described above can be combined to provide further embodiments. All of the U.S. patents, U.S. patent application publications, U.S. patent application, foreign patents, foreign patent application and non-patent publications referred to in this specification and/or listed in the Application Data Sheet are incorporated herein by reference, in their entirety. Aspects of the embodiments can be modified, if necessary to employ concepts of the various patents, application and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

The invention is also characterized by the following items:
1. A method of producing a modified B cell composition comprising:
   (a) isolating pan-B cells, memory B cells, switched memory B cells, or plasma cells from a sample, thereby obtaining an isolated B cell population,
   (b) culturing the isolated B cell population *in vitro* with one or more B cell activating factors, thereby obtaining an expanded B cell population,
   (c) transfecting the expanded B cell population with a transgene, and
   (d) differentiating the expanded B cell population *in vitro* with one or more B cell activating factors, thereby obtaining a modified B cell composition.
2. The method of item 1, wherein the transfecting step further comprises enriching the expanded B cell population using a selectable marker.
3. The method of item 2, wherein the selectable marker is selected from the group consisting of a fluorescent marker protein, a drug resistance factor, and a surface marker.
4. The method of item 1, wherein the isolated B cell population is CD20+, CD27+, and CD138-.
5. The method of item 1, wherein the isolated B cell population is CD20+ and IgG+.
6. The method of item 1, wherein the isolated B cell population is CD20-, CD38- and CD138-.
7. The method of item 1, wherein the sample is whole blood or peripheral blood mononuclear cells (PBMCs).
8. The method of item 1, wherein the isolating step comprises 1) depleting CD3+ and CD56+ cells and 2) enriching for CD27+ cells.
9. The method of item 1, wherein the one or more B cell activating factors are selected from the group consisting of CD40L, IFN-α, IFN-δ, IL-2, IL-4, IL-6, IL-10, IL-15, IL-21 and p-ODN.
10. The method of item 9, wherein CD40L is sCD40L-his.
11. The method of item 1, wherein the one or more B cell activating factors of the culturing step comprise CD40L, IL-2, IL-4 and IL-10.
12. The method of item 1, wherein the one or more B cell activating factors of the culturing step comprise CD40L, IL-2, IL-4, IL-10, IL-15 and IL-21.
13. The method of item 12, further comprising a CD40L crosslinking agent.
14. The method of item 1, wherein feeder cells are absent from the culturing step.
15. The method of item 1, wherein the expanded B cell population is migratory.
16. The method of item 15, wherein cells of the expanded B cell population migrate toward CXCL12.
17. The method of item 15, wherein at least 20% of the cells of the expanded B cell population are migratory.
18. The method of item 1, wherein the one or more B cell activating factors of the differentiating step comprise CD40L, CpG, IFN-α, IFN-δ, IL-2, IL-6, IL-10, and IL-15.
19. The method of item 1, wherein the transfecting comprises electroporation, lipofection, cell squeezing or viral transduction.
20. The method of item 19, wherein viral transduction comprises a retroviral vector.
21. The method of item 20, wherein the retroviral vector is a lentiviral vector.
22. The method of item 1, wherein the transfecting comprises a non-viral vector.
23. The method of item 22, wherein the non-viral vector is selected from the group consisting of a transposon, a zinc-finger nuclease, a transcription activator-like effector nuclease, a clustered regularly interspaced short palindromic repeat, a minicircle, a DNA replicon, an RNA replicon, an artificial chromosome, a plasmid, a mini-intronic plasmid, a nanoplasmid, a cosmid, and a bacteriophage.
24. The method of item 23, wherein the non-viral vector is a transposon.
25. The method of item 23, wherein the non-viral vector is a minicircle, a mini-intronic plasmid, or a nanoplasmid.
26. The method of item 1, wherein the transgene encodes an antigen-specific antibody, or antigen-binding fragment thereof, a fusion protein, or a therapeutic protein.
27. The method of item 26, wherein the therapeutic protein is selected from the group consisting of a cell surface receptor, a secreted protein, a signaling molecule, an antigenic fragment, an enzyme, a clotting factor, and an adhesion molecule.
28. The method of item 26, wherein the antibody is an anti-HIV antibody, an anti-RNA antibody, or an antibody that binds a protein involved in immune regulation.
29. The method of item 1, wherein cells of the modified B cell composition are CD20-, CD38+, and CD138+.
30. The method of item 1, wherein cells of the modified B cell composition are CD20-, CD38+, and CD138-.
31. A modified B cell composition produced according to the method of item 1.
32. The modified B cell composition of item 31, wherein a majority of the cells of the composition survive for 10 or more days following *in vivo* administration.
33. The modified B cell composition of item 31, wherein a majority of the cells of the composition survive for 30 or more days following *in vivo* administration.

## Claims

1. An in-vitro method of producing a modified B cell composition comprising:
(a) isolating pan-B cells from a sample, thereby obtaining an isolated B cell population,
(b) culturing the isolated B cell population *in vitro* with CD40L, IL-2, IL-4, IL-10, IL-15, IL-21, and a CD40L crosslinking agent, thereby obtaining an expanded B cell population,
(c) transfecting the expanded B cell population with a transgene, wherein the transfecting comprises electroporation of a transposon that comprises the transgene, and wherein the transgene encodes iduronidase (IDUA) for treatment or prevention of mucopolysaccharidosis type I (MPS I), and
(d) differentiating the expanded B cell population *in vitro* with one or more B cell activating factors, thereby obtaining a modified B cell composition.

2. The in-vitro method of claim 1, wherein the sample is whole blood or peripheral blood mononuclear cells (PBMCs).

3. The in-vitro method of claim 1 or 2, wherein the CD40L is sCD40L-his.

4. The in-vitro method of claim 3, wherein the CD40L cross-linking agent is an anti-poly-histidine monoclonal antibody.

5. The in-vitro method of any one of the preceding claims, wherein cells of the modified B cell composition are CD20-, CD38+, and CD138+, or wherein cells of the modified B cell composition are CD20-, CD38+, and CD138-.

6. A modified B cell composition produced according to the method of any one of claims 0-5 for use in a method of treating mucopolysaccharidosis type I (MPS I).
